# EUROPEAN PATENT APPLICATION

(11) **EP 4 756 028 A2**
(43) Date of publication of application: **10.06.2026**
(21) Application number: 25224536.0
(22) Date of filing: 15.05.2019
(51) Int. Cl.: C12N 15/113

(54) **PHARMACEUTICAL COMPOSITIONS FOR TREATMENT OF microRNA RELATED DISEASES**

(30) Priority: 18.05.2018 EP 18173161
(62) Divisional of application: 19723429.7
(71) Applicant: F. Hoffmann-La Roche AG, 4070 Basel (CH)
(72) Inventor: HENSHALL, David C., Stephen's Green D02 YN77 (IE)
(74) Representative: Rodriguez Novoa, Lorena

(57) **Abstract**

The present invention provides a pharmaceutical composition comprising an effective dosage of at least one anti miR-134 oligonucleotide for administration to a mammal suffering from a disease wherein lowering of the activity of miR-134 is beneficial, wherein single administration of the pharmaceutical composition time interval between each administration is at least 50 days and methods using such composition.

## Description

### Field of the invention

The present invention provides compositions for use in the treatment of neurological disorders such as epilepsy, and methods of treatment of said disorders, using inhibitors of microRNA-134. In particular, the invention relates to compositions comprising inhibitors of microRNA-134 for use in the treatment of said disorders (e.g. epilepsy), wherein the compositions are made for administration with a long time interval between doses, *i.e.* with a long dosage interval. The present invention further relates to methods of treatment wherein the compositions of the invention are provided to a mammal, preferably a human, and wherein the compositions are administered with a long time interval between doses.

### Background of the invention

Epilepsy is a serious, chronic neurologic disorder characterised by recurrent spontaneous seizures which affects about 50 million people worldwide and the socioeconomic cost in Europe of epilepsy is thought to be 15.5 billion euro per year (with a similar cost estimate in the U.S.A.). Anti-epileptic drugs typically control seizures in two-thirds of patients but probably do not alter the underlying pathophysiology. The remaining one-third of people with epilepsy are either drug- resistant or suffer unacceptable side effects from available drugs and continue to have seizures, leaving patients with few options, for example, brain surgery to remove part of the brain causing the seizures. The development of symptomatic (acquired) epilepsy is thought to involve altered expression of ion channels and neurotransmitter receptors, synaptic remodelling, inflammation, gliosis and neuronal death, among others. However, few anti-epileptogenic interventions targeting these processes have shown sufficient efficacy in vivo, and our understanding of the cell and molecular mechanisms remains incomplete. There is currently no prophylactic treatment ("anti-epileptogenic") following a brain injury likely to precipitate epilepsy. Similarly, there is no specific neuroprotective treatment for status epilepticus (SE), or treating acute neurolgic injuries likely to cause brain damage or epilepsy, for example, stroke, trauma. Evidence is emerging that microRNAs (miRNAs) may be critical to the pathogenesis of several neurologic disorders, including epilepsy. miRNAs are a family of small (~22nt), endogenously expressed non-coding RNAs which regulate mRNA translation by imperfect base-pairing interactions within the 3' untranslated region. Depending on the degree of sequence complementarity, miRNA binding, which occurs via Argonaute proteins within the RNA-induced silencing complex (RISC), results in either cleavage or a reduction in the translational efficiency of the target mRNA. miR-134 is a brain- specific, activity-regulated miRNA implicated in the control of neuronal microstructure. Pyramidal cells are the most common neuron in the neocortex and hippocampal formation. They are the major source of intrinsic excitatory cortical synapses, and their dendritic spines are the main postsynaptic target of excitatory synapse, with spine size and index of synaptic strength. In the adult brain, spines are quite stable but remodelling occurs during learning and memory formation, as well in the setting of neuropsychiatric disorders and pathological brain activity. Spine collapse is mediated in part by N-methdyl-D-aspartate (NMD A) receptor/calcium-dependent de- polymerisation of actin by cofilin. LIM kinase- 1 (Limkl) phosphorylates and inactivates cofilin and loss of Limkl results in abnormal spine morphology. In hippocampal neurons, miR-134 targets Limkl mRNA, thereby preventing Limkl protein translation. Over-expression of miR-134 in vitro has been reported to reduce spine volume, whereas over-expression of miR-134 in vivo using viral vectors reduces total dendritic length and abrogates long-term potentiation (LTP). Mice lacking the miRNA biogenesis component Dgrc8 fail to produce several mature miRNAs, including miR-134, and display reduced hippocampal spine density. Spine loss may have divergent functional consequences according to context, promoting excitability or uncoupling NMDA receptor-driven currents in neurons and preventing excitotoxicity.

Henshall et all in WO2013045652, assigned to the Royal College of Surgeons in Ireland describe a potential treatment or preventative measure using microRNAs against mir-134. MicroRNAs (miRNAs) are a class of small, non-coding RNAs, which work by controlling protein production at the post-transcriptional level. In the brain, miRNAs play an important role in synapse development and plasticity. Recent evidence suggests that miRNAs may play a crucial role in some forms of epilepsy. Work led by Prof. David Henshall at the Royal College of Surgeons in Ireland (RCSI), has identified a strong link between temporal lobe epilepsy (TLE) and increased expression of a miRNA, mir-134, known to play a crucial role in promoting dendrite outgrowth and negatively regulating spine maturation. Furthermore, they have shown that by silencing mir-134 in adult mouse models of *status epilepticus* using a Locked Nucleic Acid (LNA) anti miR-134 oligonucleotide, for instance antagomirs (Ant-134), they are able to powerfully suppress seizure activity and hippocampal injury caused by *status epilepticus.* Specifically, they demonstrated that treatment with antagomirs against mir-134 by ICV injection 24 hr prior to intra-amygdala kainic acid (KA)-induced *status epilepticus,* is able to significantly reduce the severity of *status epilepticus* recorded by EEG, as well as histological measurement of hippocampal damage (Jimenez-Mateos et al., 2012). Pre-treatment of mice with ICV injection of Ant-134 also potently suppressed seizures induced by the chemoconvulsants pilocarpine (Jimenez-Mateos et al, 2015) and PTZ (Reschke et al., 2017). Furthermore, antagomir (ICV) treatment 1 hr. post intra-amygdala KA injection did not reduce acute *status epilepticus* activity, but significantly delayed the onset, and reduced total number, of spontaneous seizures monitored over 2 weeks by EEG telemetry (Jimenez-Mateos et al., 2012). Remarkably, silencing miR-134 after *status epilepticus* in rats resulted in 86% reduction in the subsequent occurrence of spontaneous seizures in the perforant pathway stimulation model, a toxin-free model of acquired epilepsy (Reschke et al., 2017).

Further investigations as to an effective administration of pharmaceutical active compositions comprising therapeutically active anti miR-134 oligonucleotides designed for mammals have been conducted and the inventors have now found that such administration can surprisingly be made with a very long time interval between each administration with a near-complete cessation of seizures.

### Summary of the invention

The invention relates to a pharmaceutical composition comprising an effective dosage of at least one anti miR-134 oligonucleotide for administration to a mammal suffering from a disease wherein lowering of the activity of miR-134 is beneficial, wherein the time interval between at least two successive administrations is at least 50 days.

The invention provides for an anti miR-134 oligonucleotide for use in the treatment of a neurological disorder, such as a brain-related disorder characterized by development of seizures, wherein the time interval between two successive administrations is at least 50 days.

The invention provides for a pharmaceutical composition comprising an effective dosage of at least one anti miR-134 oligonucleotide for administration to a mammal suffering from a neurological disorder, such as a brain-related disorder characterized by development of seizures, wherein single administration of the pharmaceutical composition time interval between two successive administrations is at least 50 days.

The present invention also relates to a method of treatment of a mammal suffering from pre-existing epilepsy comprising administering a pharmaceutical composition according to the invention. The pharmaceutical composition can be administered as a single injection and wherein seizure suppression reaches up to 90% and possibly beyond and lasts for at least 50 days after injection.

The present invention provides for the use of an anti-miR-134 oligonucleotide in the manufacture of a medicament for the treatment of neurological disorder, such as a brain-related disorder characterized by development of seizures, wherein the medicament is for multiple administration to a subject wherein the time interval between two successive administrations is at least about 50 days.

In some embodiments the neurological disorder is epilepsy.

### BRIEF DESCRIPTION OF THE FIGURES

Figure 1 shows treatment with mir-134 antagomirs in adult mice with KA-induced spontaneous seizures according to a protocol described herein.
Figure 2 shows a graphic of the total number of seizures per day for experimental groups A (mice treated with saline/aCSF) , B (mice treated with mir-134 antagomir 1) and C (mice treated with mir-134 antagomir 2) according to a protocol described herein.
Figure 3 shows a graphic of the overall analysis of the total number of seizures per day for experimental groups A (n=4), B (n=3) and C (n=3) according to a protocol described herein.
Figure 4 (pictures A, B, C, D, E, F, G, H, I and J) shows representative EEG recordings traces showing differences in seizure severity between mir-134 antagomirs (e.g. group A; grey tag) and control (red tag) groups. Figure 4 (A-B) represents the occurrence of spontaneous seizures and the inter-ictal activity recording during a normal epileptic baseline period. Representative traces for "activation 1" Figure 4 (C-F) and "activation 2" Figure 4 (G-J) were obtained on the last day of recording of each respective period.

### DEFINITIONS

### Nucleotides and nucleotide analogues.

The term "nucleotide" as used herein, refers to a glycoside comprising a sugar moiety, a base moiety and a covalently linked phosphate group and covers both naturally occurring nucleotides, such as DNA or RNA, preferably DNA, and non-naturally occurring nucleotides comprising modified sugar and/or base moieties, which are also referred to as "nucleotide analogues" herein.

Non-naturally occurring nucleotides include nucleotides which have modified sugar moieties, such as bicyclic nucleotides or 2' modified nucleotides, such as 2' substituted nucleotides.

The terms "corresponding to" and "corresponds to" refer to the comparison between the nucleotide sequence of the oligomer or contiguous nucleotide sequence (a first sequence) and the equivalent contiguous nucleotide sequence of either the entire or a sub-sequence of the reverse complement of the target RNA - a oligomer sequence or contiguous nucleotide sequence thereof, which corresponds to the RNA target typically comprises no mismatches, or no more than one mismatch, when aligned to the reverse complement of the entire or a sub-sequence of the target RNA.

The expression "effective dosage" denotes the dose of a drug that will achieve the desired effect. In the context of the present invention, the desired effect is lowering of the activity of miR-134. Lowering of the activity of miR-134 can be measured by either measuring the level of miR-134, for example when using oligonucleotides which result in degradation of miR-134 or miR-134 precursors, or may be measured by measuring the de-repression of microRNA-134 targets (such as mRNAs which comprise a miR-134 binding site and whose expression is regulated by miR-134 - miR-134 target mRNAs). miR-134 inhibition may therefore be measured directly or indirectly via secondary indicators of miR-134 activity.

In this context a miR-134 target mRNA may be an endogenous target, for example a reporter gene assay, or may be an endogenous transcript whose expression is repressed by miR-134 in the absence of a miR-134 inhibitor, for example as according to methods referenced or described herein, such as, but not limited to in the examples. In one embodiment, the desired effect can be measured by the number of seizures, for example in epilepsy.

The expression "lowering the activity" means that the target mRNA is upregulated as measured by assays know in the art. An example of such assay is a commercially available assay from ThermoFisher: pMIR-REPORT miRNA Expression Reporter Vector. The pMIR-REPORT miRNA Expression Reporter Vector provides a, quantitative, "in cell" measurement of miRNA expression. This validated miRNA reporter system contains a luciferase cDNA under the control of a mammalian promoter/terminator system and a cloning region for putative miRNA binding sites in the 3' UTR. High luciferase activity indicates little or no functional interaction between the putative miRNA binding site and miRNA in the cell, while low luciferase activity indicates productive interaction between the 3' UTR sequences and miRNA in the cell.

Another example of such assays can be an in vitro assay wherein Serpine 1 is used as a biomarker for the measurement of microRNA-134 activity (see e.g. as described in EP18190523.3).

The expression "wherein lowering of the activity of miR-134 is beneficial" means that the reduction of miR-134 activity induces a benefit for a mammal suffering from a disease. This disease can for example be epilepsy.

The terms "corresponding nucleotide analogue" and "corresponding nucleotide" are intended to indicate that the nucleotide in the nucleotide analogue and the naturally occurring nucleotide are identical. For example, when the 2-deoxyribose unit of the nucleotide is linked to an adenine, the "corresponding nucleotide analogue" contains a pentose unit (different from 2-deoxyribose) linked to an adenine.

"Nucleotide analogues" are variants of natural nucleotides, such as DNA or RNA nucleotides, by virtue of modifications in the sugar and/or base moieties. Analogues could in principle be merely "silent" or "equivalent" to the natural nucleotides in the context of the oligomer, i.e. have no functional effect on the way the oligomer works to inhibit target gene expression. Such "equivalent" analogues may nevertheless be useful if, for example, they are easier or cheaper to manufacture, or are more stable to storage or manufacturing conditions, or represent a tag or label. Preferably, however, the analogues will have a functional effect on the way in which the oligomer works to inhibit expression; for example by producing increased binding affinity to the target and/or increased resistance to intracellular nucleases and/or increased ease of transport into the cell. Specific examples of nucleoside analogues are described by e.g. Freier & Altmann; Nucl. Acid Res., 1997, 25, 4429-4443 and Uhlmann; Curr. Opinion in Drug Development, 2000, 3(2), 293-213, and in Scheme 1:
**Error! Objects cannot be created from editing field codes.**

### Scheme 1

The oligomer may thus comprise or consist of a simple sequence of natural occurring nucleotides - preferably 2'-deoxynucleotides (referred to here generally as "DNA"), but also possibly ribonucleotides (referred to here generally as "RNA"), or a combination of such naturally occurring nucleotides and one or more non-naturally occurring nucleotides, i.e. nucleotide analogues. Such nucleotide analogues may suitably enhance the affinity of the oligomer for the target sequence.

Examples of suitable and preferred nucleotide analogues are provided by PCT/DK2006/000512 or are referenced therein.

Incorporation of affinity-enhancing nucleotide analogues in the oligomer, such as LNA or 2'-substituted sugars, can allow the size of the specifically binding oligomer to be reduced, and may also reduce the upper limit to the size of the oligomer before non-specific or aberrant binding takes place.

In some embodiments the oligomer or oligomers comprise at least 2 nucleotide analogues. In some embodiments, the oligomer or oligomers comprises from 3-8 nucleotide analogues, e.g. 6 or 7 nucleotide analogues. In the by far most preferred embodiments, particularly in relation to the second oligomer, but may also refer to the first oligomer, at least one of said nucleotide analogues is a locked nucleic acid (LNA); for example at least 3 or at least 4, or at least 5, or at least 6, or at least 7, or 8, of the nucleotide analogues may be LNA. In some embodiments all the nucleotides analogues may be LNA.

Examples of such modification of the nucleotide include modifying the sugar moiety to provide a 2'-substituent group or to produce a bridged (locked nucleic acid) structure which enhances binding affinity and may also provide increased nuclease resistance.

A preferred nucleotide analogue is LNA, such as oxy-LNA (such as beta-D-oxy-LNA, and alpha-L-oxy-LNA), and/or amino-LNA (such as beta-D-amino-LNA and alpha-L-amino-LNA) and/or thio-LNA (such as beta-D-thio-LNA and alpha-L-thio-LNA) and/or ENA (such as beta-D-ENA and alpha-L-ENA). Most preferred is beta-D-oxy-LNA.

In some embodiments the nucleotide analogues present within the oligomer or oligomers are independently selected from, for example: 2'-O-alkyl-RNA units, 2'-amino-DNA units, 2'-fluoro-DNA units, LNA units, arabino nucleic acid (ANA) units, 2'-fluoro-ANA units, HNA units, INA (intercalating nucleic acid -Christensen, 2002. Nucl. Acids. Res. 2002 30: 4918-4925, hereby incorporated by reference) units and 2'MOE units. In some embodiments there is only one of the above types of nucleotide analogues present in the oligomer of the invention, or contiguous nucleotide sequence thereof.

In some embodiments the nucleotide analogues are 2'-O-methoxyethyl-RNA (2'MOE), 2'-fluoro-DNA monomers or LNA nucleotide analogues, and as the oligomer or oligomers may comprise nucleotide analogues which are independently selected from these three types of analogue, or may comprise only one type of analogue selected from the three types. In some embodiments at least one of said nucleotide analogues is 2'-MOE-RNA, such as 2, 3, 4, 5, 6, 7, 8, 9 or 10 2'-MOE-RNA nucleotide units. In some embodiments at least one of said nucleotide analogues is 2'-fluoro DNA, such as 2, 3, 4, 5, 6, 7, 8, 9 or 10 2'-fluoro-DNA nucleotide units.

In some embodiments, the second oligomer comprises both LNA and 2'-MOE-RNA or 2'-fluoro nucleotides, and may, in some embodiment consist of LNA and 2'-MOE, or LNA and 2'-fluoro nucleotides.

In some embodiments, the oligomer or oligomers comprises at least one Locked Nucleic Acid (LNA) unit, such as 1, 2, 3, 4, 5, 6, 7, or 8 LNA units, such as between 3 - 7 or 4 to 8 LNA units, or 3, 4, 5, 6 or 7 LNA units. In some embodiments, all the nucleotide analogues are LNA. In some embodiments, the oligomer may comprise both beta-D-oxy-LNA, and one or more of the following LNA units: thio-LNA, amino-LNA, oxy-LNA, and/or ENA in either the beta-D or alpha-L configurations or combinations thereof. In some embodiments all LNA cytosine units are 5'methyl-Cytosine. In some embodiments of the invention, the oligomer or oligomers, may comprise both LNA and DNA units. In some embodiments, the combined total of LNA and DNA units is 10-25, or 10-20, such as 12-16. In some embodiments, the nucleotide sequence of the oligomer, such as the contiguous nucleotide sequence consists of at least one LNA and the remaining nucleotide units are DNA units. In some embodiments, the oligomer or oligomers, comprises only LNA nucleotide analogues and naturally occurring nucleotides (such as RNA or DNA, most preferably DNA nucleotides), optionally with modified internucleotide linkages such as phosphorothioate.

The term "nucleobase" refers to the base moiety of a nucleotide and covers both naturally occuring a well as non-naturally occurring variants. Thus, "nucleobase" covers not only the known purine and pyrimidine heterocycles but also heterocyclic analogues and tautomeres thereof.

Examples of nucleobases include, but are not limited to adenine, guanine, cytosine, thymidine, uracil, xanthine, hypoxanthine, 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

In some embodiments, at least one of the nucleobases present in the oligomer or oligomers is a modified nucleobase selected from the group consisting of 5-methylcytosine, isocytosine, pseudoisocytosine, 5-bromouracil, 5-propynyluracil, 6-aminopurine, 2-aminopurine, inosine, diaminopurine, and 2-chloro-6-aminopurine.

### LNA

The term "LNA" refers to a bicyclic nucleotide analogue, known as "Locked Nucleic Acid". It may refer to an LNA monomer, or, when used in the context of an "LNA oligonucleotide", LNA refers to an oligonucleotide containing one or more such bicyclic nucleotide analogues.

An "LNA nucleoside" is 2'-modified nucleoside which comprises a biradical linking the C2' and C4' of the ribose sugar ring of a nucleoside (referred also as a 2'- 4' bridge), which restricts or locks the conformation of the ribose ring. The locking of the conformation of the ribose is associated with an enhanced affinity of hybridization when the LNA is incorporated into an oligonucleotide for a complementary RNA or DNA molecule. This can be routinely determined by measuring the melting temperature of the oligonucleotide/complement duplex.

Non limiting, exemplary LNA nucleosides are disclosed in WO 99/014226, WO 00/66604, WO 98/039352 , WO 2004/046160, WO 00/047599, WO 2007/134181, WO 2010/077578, WO 2010/036698, WO 2007/090071, WO 2009/006478, WO 2011/156202, WO 2008/154401, WO 2009/067647, WO 2008/150729, Morita et al., Bioorganic & Med.Chem. Lett. 12, 73-76, Seth et al. J. Org. Chem. 2010, Vol 75(5) pp. 1569-81, and Mitsuoka et al., Nucleic Acids Research 2009, 37(4), 1225-1238.

In some embodiments, the 2' - 4' bridge is in a LNA nucleoside with a 4'-X-Y-2'biradicle (bridge), wherein....
- X is: oxygen, sulfur, -C(R^{a}R^{b})-, -C(R^{a})=C(R^{b})-, -C(R^{a})=N-, -Si(R^{a})₂-, -SO₂-, -NR^{a}- or >C=J;
- Y is: oxygen, sulfur, -C(R^{a}R^{b})-, -C(R^{a})=C(R^{b})-, -C(R^{a}R^{b})-C(R^{a}R^{b})-,-C(R^{a}R^{b})C(R^{a}R^{b})C(R^{a}R^{b})-, -C(R^{a})=N-, -Si(R^{a})₂-, -SO₂-, -N(R^{a})- or >C=J; wherein J is oxygen, sulfur or =N(R^{a});

with the proviso that -X-Y- is other than -O-O-; Si(R^{a})2-Si(R^{a})2-; -SO₂-SO₂-;-C(R^{a})=C(R^{b})-C(R^{a})=C(R^{b}); -C(R^{a})=N-C(R^{a})=N; -C(R^{a})=N-C(R^{a})=C(R^{b}); and-C(R^{a})=C(R^{b}) C(R^{a})=N -,
or -X-Y- together designate -OC(R^{a} R^{b})-, -SC(R^{a} R^{b})-, -NR^{a}C(R^{a} R^{b})-, -OCR^{a}R^{b} -,-CR^{a}R^{b}OCR^{a}R^{b} -, -OC R^{a}R^{b}CR^{a}R^{b}-, -CHR^{a}OCHR^{b} -, -OCHR^{a}CHR^{b}-, -OCR^{a}CR^{b}-, -OC(R^{a} R^{b})C(R^{a} R^{b})C(R^{a} R^{b})-, -OC(R^{a} R^{b})OC(R^{a} R^{b})-, -ONR^{a}C(R^{a} R^{b})-,-C(=CR^{a}R^{b})C(R^{a} R^{b})-, -NR^{a}C(R^{a} R^{b})-, -R^{a}NOC(R^{a} R^{b})- or -SC(R^{a} R^{b});
wherein R^{a} and R^{b} are independently selected from hydrogen, halogen, hydroxyl, a protecting group, C₁₋₆-alkyl, substituted C₁₋₆-alkyl, C₂₋₆-alkenyl, substituted C₂₋₆-alkenyl, C₂₋₆-alkynyl, substituted C₂₋₆-alkynyl, C₁₋₆-alkoxy, substituted C₁₋₆-alkoxy, C₂₋₆-alkoxyalkyl, C₂₋₆-alkenyloxy, carboxy, C₁₋₆-alkoxycarbonyl, C₁₋₆-alkylcarbonyl, formyl, C_{5 - 8} aryl, substituted C_{5 - 8} aryl, amino, mono- and di(C₁₋₆-alkyl)amino, carbamoyl, mono- and di(C₁₋₆-alkyl)-amino-carbonyl, amino-C₁₋₆-alkyl-aminocarbonyl, mono- and di(C₁₋₆-alkyl)amino-C₁₋₆-alkyl-aminocarbonyl, C₁₋₆-alkyl-carbonylamino, carbamido, C₁₋₆-alkanoyloxy, sulphono, C₁₋₆-alkylsulphonyloxy, nitro, azido, sulphanyl, C₁₋₆-alkylthio, C_{5 - 8} aryloxy-carbonyl, C_{5 - 8} aryloxy, C_{5 - 8} arylcarbonyl, C_{5 - 8} heteroaryl, C_{5 - 8} heteroaryloxy-carbonyl, *C*_{5 - 8} heteroaryloxy, C_{5 - 8} heteroarylcarbonyl, -OR^{c},-NR^{c}R^{d}, -SR^{c}, -N3, -OC(=X^{a})R^{c}, -OC(=X^{a})NR^{c}R^{d}, -NR^{e}C(=X^{a})NR^{c}R^{d} and -CN, wherein each R^{c}, R^{d} and R^{e} is, independently, H or C₁₋₆ alkyl, and X^{a} is O, S or NR^{c}, -C(=O)-R^{a}, or the two geminal substituents R^{a} and R^{b} together may designate methylene (=CH₂), or substituted methylene; wherein when substituted R^{a} and R^{b} are independently, mono or poly substituted with halogen, hydroxyl, a protecting group, C₁₋₆-alkyl, C₂₋₆-alkenyl, C₂₋₆-alkynyl, C₁₋₆-alkoxy, C₂₋₆-alkoxyalkyl, C₂₋₆-alkenyloxy, carboxy, C₁₋₆-alkoxycarbonyl, C₁₋₆-alkylcarbonyl, formyl, C_{5 - 8} aryl, or C_{5 - 8} heteroaryl.

In some embodiments, R^{a} and R^{b} are independently selected from the group consisting of hydrogen, halogen, hydroxyl, C₁₋₆ alkyl and C₂₋₆-alkoxyalkyl.

In some embodiments, R^{a} and R^{b} are independently selected from the group consisting of hydrogen, halogen, hydroxyl, methyl and CH₂OCH₃.

In some embodiments, R^{a} and R^{b} are independently selected from the group consisting of hydrogen, methyl and CH₂OCH₃.

In some embodiments, R^{a} is hydrogen or methyl.

In some embodiments, R^{b} is hydrogen or methyl.

In some embodiments, one or both of R^{a} and R^{b} are hydrogen.

In some embodiments, only one of R^{a} and R^{b} is hydrogen.

In some embodiments, one of R^{a} and R^{b} is methyl and the other one is hydrogen.

In some embodiments, R^{a} and R^{b} are both methyl at the same time.

In some embodiments, X is oxygen, sulfur, NH-, -CH₂- or -C(=CH₂)-.

In some embodiments, X is oxygen.

In some embodiments, Y is -CH₂-, -CHCH₃-, -CH₂CH₂- or -CH₂CH₂CH₂-.

In some embodiments, -X-Y- is -OCH₂- or -OCH(CH₃)-.

In some embodiments -X-Y- is -O-CR^{a}R^{b-} wherein R^{a} and R^{b} are independently selected from the group consisting of hydrogen, methyl and CH₂OCH₃.

In some embodiments -X-Y- is -O-CR^{a}R^{b-} wherein one of R^{a} and R^{b} is hydrogen, and the other is selected from the group consisting of methyl and CH₂OCH₃.

In some embodiments -X-Y- is -O-CH₂⁻.

### Further Non-limiting Examples of LNA Nucleosides

The 2' - 4' bridge may be positioned either below the plane of the ribose ring (beta-D-configuration), or above the plane of the ring (alpha-L- configuration), as illustrated in formula (A) and formula (B) respectively:

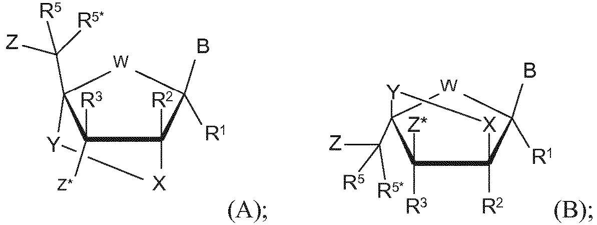

It will be understood that the LNA nucleosides illustrates in formula A and B are for illustrative purposes.

In some embodiments, the LNA nucleoside is a nucleoside of formula A, wherein -X-Y- is as defined above.

In some embodiments, the LNA nucleoside is a nucleoside of formula B, wherein -X-Y- is as defined above.
wherein
W is oxygen, sulfur, -N(R^{a})- or -C(R^{a}R^{b})-, in particular oxygen; wherein R^{a} and R^{b} are as defined according to -X- or -Y- herein;
B is a nucleobase or a modified nucleobase;
Z is an internucleoside linkage to an adjacent nucleoside or a 5'-terminal group;
Z* is an internucleoside linkage to an adjacent nucleoside or a 3'-terminal group;
R¹, R², R³, R⁵ and R^{5*} are independently selected from hydrogen, C₁₋₆-alkyl, C₂₋₆-alkenyl, C₂₋₆-alkynyl, hydroxy, C₁₋₆-alkoxy, C₂₋₆-alkoxyalkyl, C₂₋₆-alkenyloxy, carboxy, C₁₋₆-alkoxycarbonyl, C₁₋₆-alkylcarbonyl, formyl, and C₅₋₈ aryl;

In some embodiments R¹, R², R³, R⁵ and R^{5*} are independently selected from C₁₋₆ alkyl, such as methyl, and hydrogen.

In some embodiments R¹, R², R³, R⁵ and R^{5*} are all hydrogen at the same time.

In some embodiments R¹, R², R³, are all hydrogen at the same time, one of R⁵ and R^{5*} is hydrogen and the other of R⁵ and R^{5*} is as defined above, in particular C₁₋₆ alkyl, such as methyl.

In some embodiments, -X-Y- is -OCH₂-, W is oxygen and R¹, R², R³, R⁵ and R^{5*} are all hydrogen at the same time. Such LNA nucleosides are disclosed in WO 99/014226, WO 00/66604, WO 98/039352 and WO 2004/046160 which are all hereby incorporated by reference, and include what are commonly known in the art as beta-D-oxy LNA and alpha-L-oxy LNA nucleosides.

In some embodiments, -X-Y- is -SCH₂-, W is oxygen and R¹, R², R³, R⁵ and R^{5*} are all hydrogen at the same time. Such thio LNA nucleosides are disclosed in WO 99/014226 and WO 2004/046160 which are hereby incorporated by reference.

In some embodiments, -X-Y- is -NHCH₂-, W is oxygen and R¹, R², R³, R⁵ and R^{5*} are all hydrogen at the same time. Such amino LNA nucleosides are disclosed in WO 99/014226 and WO 2004/046160 which are hereby incorporated by reference.

In some embodiments, -X-Y- is -OCH₂CH₂- or -OCH₂CH₂CH₂-, W is oxygen, and R¹, R², R³, R⁵ and R^{5*} are all hydrogen at the same time. Such LNA nucleosides are disclosed in WO 00/047599 and Morita et al., Bioorganic & Med.Chem. Lett. 12, 73-76, which are hereby incorporated by reference, and include what are commonly known in the art as 2'-O-4'C-ethylene bridged nucleic acids (ENA).

In some embodiments, -X-Y- is -OCH₂-, W is oxygen, R¹, R², R³ are all hydrogen at the same time, one of R⁵ and R^{5*} is hydrogen and the other one is not hydrogen, such as C₁₋₆ alkyl, for example methyl. Such 5' substituted LNA nucleosides are disclosed in WO 2007/134181 which is hereby incorporated by reference.

In some embodiments, -X-Y- is -OCR^{a}R^{b}-, wherein one or both of R^{a} and R^{b} are other than hydrogen, such as methyl, W is oxygen, R¹, R², R³ are all hydrogen at the same time, one of R⁵ and R^{5*} is hydrogen and the other one is not hydrogen, such as C₁₋₆ alkyl, for example methyl. Such bis modified LNA nucleosides are disclosed in WO 2010/077578 which is hereby incorporated by reference.

In some embodiments, -X-Y- is -O-CH(CH₂OCH₃)- (2' O-methoxyethyl bicyclic nucleic acid - Seth et al. J. Org. Chem. 2010, Vol 75(5) pp. 1569-81).

In some embodiments, -X-Y- is -O-CH(CH₂CH₃)- (2'O-ethyl bicyclic nucleic acid - Seth at al., J. Org. Chem. 2010, Vol 75(5) pp. 1569-81).

In some embodiments, -X-Y- is -O-CHR^{a}-, W is oxygen and R¹, R², R³, R⁵ and R^{5*} are all hydrogen at the same time. Such 6' substituted LNA nucleosides are disclosed in WO 2010/036698 and WO 2007/090071 which are both hereby incorporated by reference.

In some embodiments, -X-Y- is -OCH(CH₂OCH₃)-, W is oxygen and R¹, R², R³, R⁵ and R^{5*} are all hydrogen at the same time. Such LNA nucleosides are also known in the art as cyclic MOEs (cMOE) and are disclosed in WO 2007/090071.

In some embodiments, -X-Y- is -OCH(CH₃)-, in either (R) or (S) configuration.

In some embodiments, -X-Y- is -OCH₂OCH₂- (Seth et al., J. Org. Chem 2010 op. cit.)

In some embodiments, -X-Y- is -OCH(CH₃)-, W is oxygen and R¹, R², R³, R⁵ and R^{5*} are all hydrogen at the same time. Such 6' methyl LNA nucleosides are also known in the art as cET nucleosides, and may be either (S)-cET or (R)-cET enantiomers, as disclosed in WO 2007/090071 (beta-D) and WO 2010/036698 (alpha-L) which are both hereby incorporated by reference.

In some embodiments, -X-Y- is -O-CR^{a}R^{b}-, wherein neither R^{a} nor R^{b} is hydrogen, W is oxygen, and R¹, R², R³, R⁵ and R^{5*} are all hydrogen at the same time. In some embodiments, R^{a} and R^{b} are both methyl. Such 6' di-substituted LNA nucleosides are disclosed in WO 2009/006478 which is hereby incorporated by reference.

In some embodiments, the -X-Y- is -SCHR^{a}-, W is oxygen, and R¹, R², R³, R⁵ and R^{5*} are all hydrogen at the same time. Such 6' substituted thio LNA nucleosides are disclosed in WO 2011/156202 which is hereby incorporated by reference. In some 6' substituted thio LNA embodiments R^{a} is methyl.

In some embodiments, -X-Y- is -C(=CH₂)C(R^{a}R^{b})-, such as -C(=CH₂)CH₂- , or-C(=CH₂)CH(CH₃)-, W is oxygen, and R¹, R², R³, R⁵ and R^{5*} are all hydrogen at the same time. Such vinyl carbo LNA nucleosides are disclosed in WO 2008/154401 and WO 2009/067647 which are both hereby incorporated by reference.

In some embodiments, -X-Y- is -N(-OR^{a})-, W is oxygen and R¹, R², R³, R⁵ and R^{5*} are all hydrogen at the same time. In some embodiments, R^{a} is C₁₋₆ alkyl such as methyl. Such LNA nucleosides are also known as N substituted LNAs and are disclosed in WO 2008/150729 which is hereby incorporated by reference.

In some embodiments, -X-Y- is -ONR^{a}CH₃- (Seth et al., J. Org. Chem 2010 op. cit.)

In some embodiments, -X-Y- is -N(R^{a})-, W is oxygen, and R¹, R², R³, R⁵ and R^{5*} are all hydrogen at the same time. In some embodiments, R^{a} is C₁₋₆ alkyl, such as methyl.

In some embodiments, R⁵ and R^{5*} are both hydrogen at the same time. In some embodiments, one of R⁵and R^{5*} is hydrogen and the other one is C₁₋₆ alkyl, such as methyl. In such an embodiment, R¹, R² and R³ may all be hydrogen, and -X-Y- may be -OCH₂- or-OCHC(R^{a})₃-, such as -OCH(CH₃)-.

In some embodiments, -X-Y- is -CR^{a}R^{b}-O-CR^{a}R^{b}-, such as -CH₂OCH₂-, W is oxygen and R¹, R², R³, R⁵ and R^{5*} are all hydrogen at the same time. In some embodiments R^{a} is C₁₋₆ alkyl such as methyl. Such LNA nucleosides are also known as conformationally restricted nucleotides (CRNs) and are disclosed in WO 2013/036868 which is hereby incorporated by reference.

In some embodiments, -X-Y- is -O-CR^{a}R^{b}-O-CR^{a}R^{b}-, such as -OCH₂OCH₂-, W is oxygen and R¹, R², R³, R⁵ and R^{5*} are all hydrogen at the same time. In some embodiments R^{a} is C₁₋₆ alkyl such as methyl. Such LNA nucleosides are also known as COC nucleotides and are disclosed in Mitsuoka et al., Nucleic Acids Research 2009, 37(4), 1225-1238, which is hereby incorporated by reference.

It will be recognized than, unless specified, the LNA nucleosides may be in the beta-D or alpha-L stereoisoform.

Particular examples of LNA nucleosides are presented in Scheme 1.

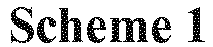

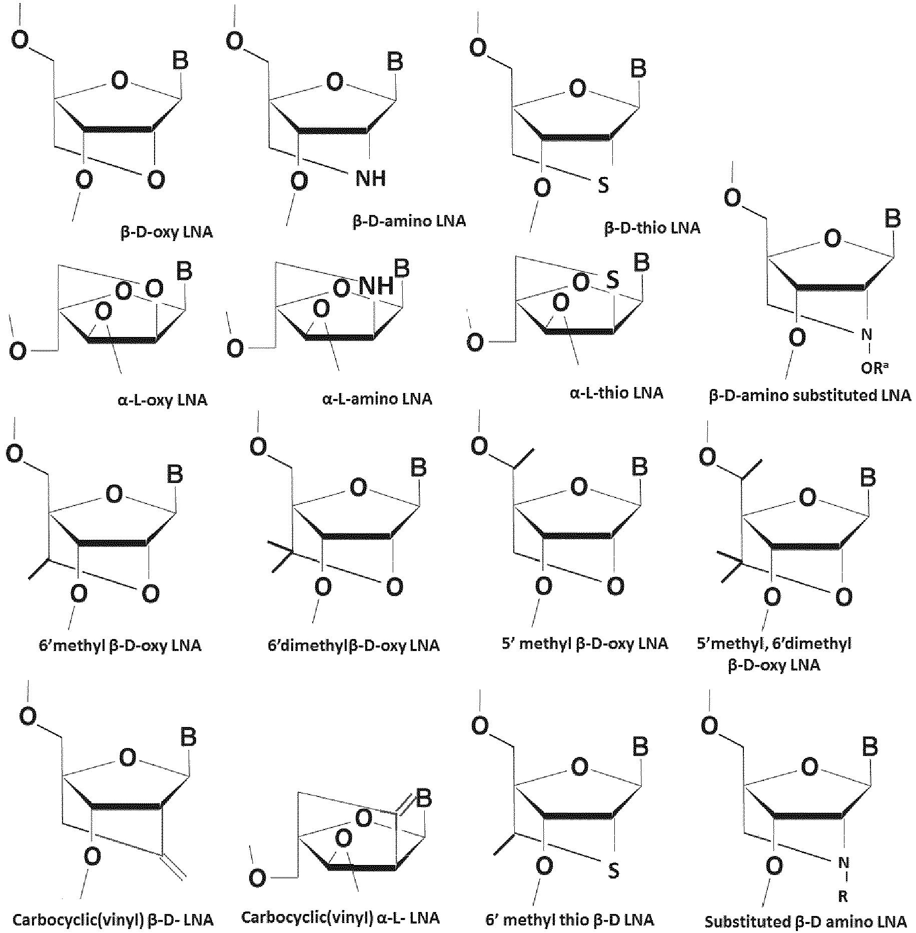

Particular LNA nucleosides are beta-D-oxy-LNA, 6'methyl beta-D-oxy LNA and ENA.

### Internucleotide Linkages

The terms "linkage group" or "internucleotide linkage" are intended to mean a group capable of covalently coupling together two nucleotides, two nucleotide analogues, and a nucleotide and a nucleotide analogue, etc. Specific and preferred examples include phosphate groups and phosphorothioate groups.

The nucleotides of the oligomer of the invention or contiguous nucleotides sequence thereof are coupled together via linkage groups. Suitably each nucleotide is linked to the 3' adjacent nucleotide via a linkage group.

Suitable internucleotide linkages include those listed within PCT/DK2006/000512, for example the internucleotide linkages listed on the first paragraph of page 34 of PCT/DK2006/000512 (hereby incorporated by reference).

It is, in some embodiments, preferred to modify the internucleotide linkage from its normal phosphodiester to one that is more resistant to nuclease attack, such as phosphorothioate or boranophosphate - these two, being cleavable by RNase H, also allow that route of antisense inhibition in reducing the expression of the target gene.

Suitable sulphur (S) containing internucleotide linkages as provided herein may be preferred. Phosphorothioate internucleotide linkages are also preferred.

The internucleotide linkages in the oligomer may be phosphodiester, phosphorothioate or boranophosphate. Phosphorothioate is preferred, for improved nuclease resistance and other reasons, such as ease of manufacture.

In one aspect of the oligomer of the invention, the nucleotides and/or nucleotide analogues are linked to each other by means of phosphorothioate groups.

It is recognised that the inclusion of phosphodiester linkages, such as one or two linkages, into an otherwise phosphorothioate oligomer, particularly between or adjacent to nucleotide analogue units can modify the bioavailability and/or bio-distribution of an oligomer - see WO2008/053314, hereby incorporated by reference.

In some embodiments, such as the embodiments referred to above, where suitable and not specifically indicated, all remaining linkage groups are either phosphodiester or phosphorothioate, or a mixture thereof.

In some embodiments all the internucleotide linkage groups are phosphorothioate.

When referring to specific gapmer oligonucleotide sequences, such as those provided herein it will be understood that, in various embodiments, when the linkages are phosphorothioate linkages, alternative linkages, such as those disclosed herein may be used, for example phosphate (phosphodiester) linkages may be used, particularly for linkages between nucleotide analogues, such as LNA, units.

### Conjugates

In the context the term "conjugate" is intended to indicate a heterogenous molecule formed by the covalent attachment ("conjugation") of the oligomer as described herein to one or more non-nucleotide, or non-polynucleotide moieties. Examples of non-nucleotide or non-polynucleotide moieties include macromolecular agents such as proteins, fatty acid chains, sugar residues, glycoproteins, polymers, or combinations thereof. Typically proteins may be antibodies for a target protein. Typical polymers may be polyethylene glycol.

Therefore, in various embodiments, the oligomer of the invention may comprise both a polynucleotide region which typically consists of a contiguous sequence of nucleotides, and a further non-nucleotide region. When referring to the oligomer of the invention consisting of a contiguous nucleotide sequence, the compound may comprise non-nucleotide components, such as a conjugate component.

In various embodiments of the invention the oligomeric compound is linked to ligands/conjugates, which may be used, e.g. to increase the cellular uptake of oligomeric compounds. WO2007/031091 provides suitable ligands and conjugates, which are hereby incorporated by reference.

The invention also provides for a conjugate comprising the compound according to the invention as herein described, and at least one non-nucleotide or non-polynucleotide moiety covalently attached to said compound. Therefore, in various embodiments where the compound of the invention consists of a specified nucleic acid or nucleotide sequence, as herein disclosed, the compound may also comprise at least one non-nucleotide or non-polynucleotide moiety (e.g. not comprising one or more nucleotides or nucleotide analogues) covalently attached to said compound.

Conjugation (to a conjugate moiety) may enhance the activity, cellular distribution or cellular uptake of the oligomer of the invention. Such moieties include, but are not limited to, antibodies, polypeptides, lipid moieties such as a cholesterol moiety, cholic acid, a thioether, e.g. Hexyl-s-tritylthiol, a thiocholesterol, an aliphatic chain, e.g., dodecandiol or undecyl residues, a phospholipids, e.g., di-hexadecyl-rac-glycerol or triethylammonium 1,2-di-o-hexadecyl-rac-glycero-3-h-phosphonate, a polyamine or a polyethylene glycol chain, an adamantane acetic acid, a palmityl moiety, an octadecylamine or hexylamino-carbonyl-oxycholesterol moiety.

The oligomers of the invention may also be conjugated to active drug substances, for example, aspirin, ibuprofen, a sulfa drug, an antidiabetic, an antibacterial or an antibiotic.

In certain embodiments the conjugated moiety is a sterol, such as cholesterol.

In various embodiments, the conjugated moiety comprises or consists of a positively charged polymer, such as a positively charged peptides of, for example between 1 -50, such as 2 - 20 such as 3 - 10 amino acid residues in length, and/or polyalkylene oxide such as polyethylglycol(PEG) or polypropylene glycol - see WO 2008/034123, hereby incorporated by reference. Suitably the positively charged polymer, such as a polyalkylene oxide may be attached to the oligomer of the invention via a linker such as the releasable inker described in WO 2008/034123.

By way of example, the following conjugate moieties may be used in the conjugates of the invention:

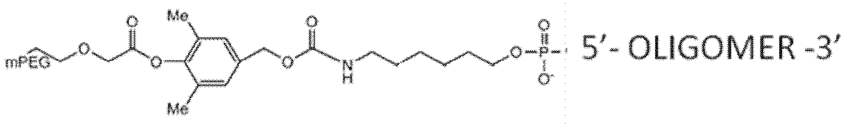

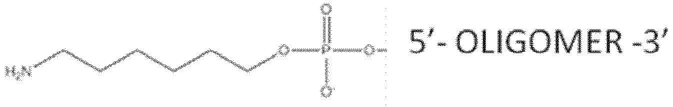

### Activated oligomers

The term "activated oligomer," as used herein, refers to an oligomer of the invention that is covalently linked (i.e., functionalized) to at least one functional moiety that permits covalent linkage of the oligomer to one or more conjugated moieties, i.e., moieties that are not themselves nucleic acids or monomers, to form the conjugates herein described. Typically, a functional moiety will comprise a chemical group that is capable of covalently bonding to the oligomer via, e.g., a 3'-hydroxyl group or the exocyclic NH₂ group of the adenine base, a spacer that is preferably hydrophilic and a terminal group that is capable of binding to a conjugated moiety (e.g., an amino, sulfhydryl or hydroxyl group). In some embodiments, this terminal group is not protected, e.g., is an NH₂ group. In other embodiments, the terminal group is protected, for example, by any suitable protecting group such as those described in "Protective Groups in Organic Synthesis" by Theodora W. Greene and Peter G M Wuts, 3rd edition (John Wiley & Sons, 1999). Examples of suitable hydroxyl protecting groups include esters such as acetate ester, aralkyl groups such as benzyl, diphenylmethyl, or triphenylmethyl, and tetrahydropyranyl. Examples of suitable amino protecting groups include benzyl, alpha-methylbenzyl, diphenylmethyl, triphenylmethyl, benzyloxycarbonyl, tert-butoxycarbonyl, and acyl groups such as trichloroacetyl or trifluoroacetyl. In some embodiments, the functional moiety is self-cleaving. In other embodiments, the functional moiety is biodegradable. See e.g., U.S. Patent No. 7,087,229, which is incorporated by reference herein in its entirety.

In some embodiments, oligomers of the invention are functionalized at the 5' end in order to allow covalent attachment of the conjugated moiety to the 5' end of the oligomer. In other embodiments, oligomers of the invention can be functionalized at the 3' end. In still other embodiments, oligomers of the invention can be functionalized along the backbone or on the heterocyclic base moiety. In yet other embodiments, oligomers of the invention can be functionalized at more than one position independently selected from the 5' end, the 3' end, the backbone and the base.

In some embodiments, activated oligomers of the invention are synthesized by incorporating during the synthesis one or more monomers that is covalently attached to a functional moiety. In other embodiments, activated oligomers of the invention are synthesized with monomers that have not been functionalized, and the oligomer is functionalized upon completion of synthesis. In some embodiments, the oligomers are functionalized with a hindered ester containing an aminoalkyl linker, wherein the alkyl portion has the formula (CH₂)_{w}, wherein w is an integer ranging from 1 to 10, preferably about 6, wherein the alkyl portion of the alkylamino group can be straight chain or branched chain, and wherein the functional group is attached to the oligomer via an ester group (-O-C(O)-(CH₂)_{w}NH).

In other embodiments, the oligomers are functionalized with a hindered ester containing a (CH₂)_{w}-sulfhydryl (SH) linker, wherein w is an integer ranging from 1 to 10, preferably about 6, wherein the alkyl portion of the alkylamino group can be straight chain or branched chain, and wherein the functional group attached to the oligomer via an ester group (-O-C(O)-(CH₂)_{w}SH)

In some embodiments, sulfhydryl-activated oligonucleotides are conjugated with polymer moieties such as polyethylene glycol or peptides (via formation of a disulfide bond).

Activated oligomers containing hindered esters as described above can be synthesized by any method known in the art, and in particular by methods disclosed in PCT Publication No. WO 2008/034122 and the examples therein, which is incorporated herein by reference in its entirety.

In still other embodiments, the oligomers of the invention are functionalized by introducing sulfhydryl, amino or hydroxyl groups into the oligomer by means of a functionalizing reagent substantially as described in U.S. Patent Nos. 4,962,029 and 4,914,210, i.e., a substantially linear reagent having a phosphoramidite at one end linked through a hydrophilic spacer chain to the opposing end which comprises a protected or unprotected sulfhydryl, amino or hydroxyl group. Such reagents primarily react with hydroxyl groups of the oligomer. In some embodiments, such activated oligomers have a functionalizing reagent coupled to a 5'-hydroxyl group of the oligomer. In other embodiments, the activated oligomers have a functionalizing reagent coupled to a 3'-hydroxyl group. In still other embodiments, the activated oligomers of the invention have a functionalizing reagent coupled to a hydroxyl group on the backbone of the oligomer. In yet further embodiments, the oligomer of the invention is functionalized with more than one of the functionalizing reagents as described in U.S. Patent Nos. 4,962,029 and 4,914,210, incorporated herein by reference in their entirety. Methods of synthesizing such functionalizing reagents and incorporating them into monomers or oligomers are disclosed in U.S. Patent Nos. 4,962,029 and 4,914,210.

In some embodiments, the 5'-terminus of a solid-phase bound oligomer is functionalized with a dienyl phosphoramidite derivative, followed by conjugation of the deprotected oligomer with, e.g., an amino acid or peptide via a Diels-Alder cycloaddition reaction.

In various embodiments, the incorporation of monomers containing 2'-sugar modifications, such as a 2'-carbamate substituted sugar or a 2'-(O-pentyl-N-phthalimido)-deoxyribose sugar into the oligomer facilitates covalent attachment of conjugated moieties to the sugars of the oligomer. In other embodiments, an oligomer with an amino-containing linker at the 2'-position of one or more monomers is prepared using a reagent such as, for example, 5'-dimethoxytrityl-2'-O-(e-phthalimidylaminopentyl)-2'-deoxyadenosine-3'-- N,N-diisopropyl-cyanoethoxy phosphoramidite. See, e.g., Manoharan, et al., Tetrahedron Letters, 1991, 34, 7171.

In still further embodiments, the oligomers of the invention may have amine-containing functional moieties on the nucleobase, including on the N6 purine amino groups, on the exocyclic N2 of guanine, or on the N4 or 5 positions of cytosine. In various embodiments, such functionalization may be achieved by using a commercial reagent that is already functionalized in the oligomer synthesis.

Some functional moieties are commercially available, for example, heterobifunctional and homobifunctional linking moieties are available from the Pierce Co. (Rockford, Ill.). Other commercially available linking groups are 5'-Amino-Modifier C6 and 3'-Amino-Modifier reagents, both available from Glen Research Corporation (Sterling, Va.). 5'-Amino-Modifier C6 is also available from ABI (Applied Biosystems Inc., Foster City, Calif.) as Aminolink-2, and 3'-Amino-Modifier is also available from Clontech Laboratories Inc. (Palo Alto, Calif.).

In the context of this patent application, microRNA's (miRNAs) are small (~22nt) non-coding RNAs (ncRNAs) that regulate gene expression at the level of translation. Each miRNA apparently regulates multiple genes and hundreds of miRNA genes are predicted to be present in mammals. Recently miRNAs have been found to be critical for development, cell proliferation and cell development, apoptosis and fat metabolism, and cell differentiation.

In the specification, the term "miR-134" should be understood to mean a brain- specific, activity-regulated miRNA implicated in the control of neuronal micro structure. Pyramidal cells are the most common neuron in the neocortex and hippocampal formation. They are the major source of intrinsic excitatory cortical synapses, and their dendritic spines are the main postsynaptic target of excitatory synapse, with spine size and index of synaptic strength. In the adult brain, spines are quite stable but remodeling occurs during learning and memory formation, as well in the setting of neuropsychiatric disorders and pathological brain activity. Over-expression of miR-134 in vitro has been reported to reduce spine volume, whereas over-expression of miR-134 in vivo using viral vectors reduces total dendritic length and abrogates long-term potentiation (LTP). Mature human miR-134 (hsa-miR-134) has the following sequence SEQ ID NO: 1: UGUGACUGGUUGACCAGAGGGG. (the seed region is underlined). Advantageously the target nucleic acis may be hsa-miR-134).

The primary sequence of human miR-134 is provided below:
SEQ ID NO: 2:
GGACCACAATTTCAACTCCAGGGAAGGGGGACTGTGCCAGCACCACTCCAAGG GAGGTGAGTGAAGGGTTGCCCAAACTCCAGTGTGTTCCTAGGACACCCGTAAG CTGCCTCACTAATGCTCGGTGTCCACTCTGTCCACAGGATGGTGGTTGGCAGCC ACTCCCCTTGGAGAAGTGGAAGGGGACTCCTTGTCTGTCTTGTCTCTGCTTTTC TGTGGTACTTGAAGAGAAGTTGTTCGTGGTGGATTCGCTTTACTTATGACGAAT CATTCACGGACAACACTTTTTTCAGTACCAAATGCTACCTCTAAGGACTTCCTG GACACAATGGCAGCTTCAGGAAAGATAGTCTTTGTGTCAACCATGTGGAAAAG CCAAGAATGGATGGCGGGCCATGGACAATGCGCTGACCTAGCTGTAAGTCACC TGGCCCGATAATCCGAGCCTCCCATGCACCTATAGGAGGTCTTCCCATGGGTCT CACCAACTCTGGGGAATCAGCTGTGGTTCTGTCACCAGCGTCACCTCACAAGA CTTTGAAGAGAGGCTCCCTGGGCCCCAGGCCGACTTCCAGAAGAGATGTTGGT GTCAGCACCGTCCAGGGTGTGTGACTGGTTGACCAGAGGGGCATGCACTG TGTTCACCCTGTGGGCCACCTAGTCACCAACCCTCAGCATCACTCCCACTCC AGGAAGACTTTCCAGAGCTCCCACCAACTCTGGGGAAGCGGCCATGGACTTGC TGTGACTGCTTGGTGGACTGCGATGACACGTGCTCTTGGGGGTGTATGTTTACT TAAAATGCAATGAGTCAGCCTTGGCAGCCCCTTCACCACTGTGACAGCCTCCTT GAAGTGTTGACTTCCGATGTGGGACGCCATGTTGTCTTCTGTTGAGGGACCTGA TGTGGGCCAGCTTTTCTCCTGGGTGTGTGACTGAATCCTTCTTCCCAGAGCATG TGCTGCCTTTGTTAGGTCTGTCACGTCGTCCTCTAATACCCAGCATCCTGTCTCT CCCTAGGAGGCTCCATGGAGATAATGCGGCTTTGGGAAGCGGCCATGATTCTC CTGTCAGGGACCAGTGAGCTACGCAAAAGCTCCCTGTCTTGTCTGGAAGGACG AACTGCATCCTTGCTGCTGGGGAGAAGGCAGTGCCCTCAGCACTCCCTTAAGG TAAGTGCGCCTCGGGTGAGCATGCACTTAATGTGGGTGTATGTCACTCGGCTC GGCCCACTACCCAATACTATCCCACCCATTCCTAACAGGACTCCCGA. The precursor sequence of human miR-134 is is as following: SEQ ID NO: 3:
CAGGGTGTGTGACTGGTTGACCAGAGGGGCATGCACTGTGTTCACCCTGTG GGCCACCTAGTCACCAACCCTC.

The term "human miR- 134" should also be taken to include any human miR-134 paralogues.

In the specification, the term "inhibition of miR- 134 activity" should be understood to mean preventing miR-134 carrying out its function(s), typically including controlling neuronal microstructure. Generally, inhibiting miR-134 activity should be understood to include direct inhibition in which a molecule binds to miR-134 and directly inhibits its activity (for example, a low molecular weight inhibitor or a binding partner such as an antibody or antibody fragment) and indirect inhibition in which, for example, expression of the miRNA molecule is modulated by suitable means including for example use of repressors or small interfering RNA molecules. Inhibition of miR-134 function should be understood to encompass administering any molecule which directly or indirectly inhibits the mature form of miR- 134, as well as molecules which target the precursor form of miR-134, primary miR-134, or any human miR-134 paralogues. The term should also include administering molecules which interfere with miR-134 function by enhancing expression of its target mRNA transcripts, including but not limited to Limkl, and molecules which target the biogenesis of miR-134 and might thereby be capable of inhibiting production of miR- 134 such as molecules targeting the transcription regulation of miR-134 or other steps in miR-134 biogenesis (for instance, molecules that inhibit Argonaute-2, Drosha/DGCR8 or Dicer). The antisense targeting region of miR-134 is defined by: SEQ ID NO: 4: UCUCUCCCUCUGGUCAACCAGUCACAAGGCU. Thus, the agent capable of inhibiting miR-134 activity may be a molecule capable of specifically binding to the antisense targeting region of miR- 134 as shown above. An example of such an agent is a reverse complementarity sequence (e.g. as an oligo or antisense nucleotide) as following: SEQ ID NO: 5: CCCCTCTGGTCAACCAGTCACA .

In the specification, the term "agent capable of inhibiting miR- 134 activity" or "anti miR-134" are used interchangeably and should be understood to mean any agent which may inhibit, ideally specifically inhibit, the expression of or activity miR- 134.

Suitable agents include antisense molecules (such as antagomirs), small hairpin RNA molecules, small interfering RNA molecules, microRNA sponges, tiny seed- targeting locked nucleic acid (LNA) oligonucleotides, decoy oligonucleotides, aptamers, ribozymes, or antibodies that specifically recognize DNA:RNA heteroduplexes. Small hairpin RNA (shRNA) molecules are short RNA molecules having a small hairpin loop in their tertiary structure that may be employed to silence genes. The design of shRNA molecules capable of inhibiting miR-134 will be apparent to those skilled in the field of shRNA molecule design. As an alternative, the level of miR-134 expression can be modulated using antisense or ribozyme approaches to inhibit or prevent miR-134 activity, or triple helix approaches to inhibit transcription of miR-134.

An advantageous anti-miR 134 agent is an antisense oligonucleotide. The term an "anti miR-134 oligonucleotide" refers to an oligonucleotide which is complementary to a microRNA-134 target nucleic acid and is capable of hybridizing to and inhibiting the activity of the microRNA-134 target nucleic acid. The term antagomir as used herein is used generally to refer to anti-miR-134 oligonucleotides which target the mature microRNA, such as hsa-miR-134. An advantageous antagomir for use in the present invention is a LNA antimiR.

In the specification, the term "brain-related disorder characterized by development of seizures" should be understood to mean a brain injury or condition that can precipitate seizures or epilepsy, such as for example, stroke, CNS infection-associated seizures, brain tumors, traumatic brain injury, neurodegenerative disorders, metabolic disorders causing seizures (including but not limited to hypoglycaemia, glycogen storage diseases, pyruvate dehydrogenase deficiency, acquired hypoparathyroidism, Adenylosuccinate lyase (ADSL) deficiency) and autoimmune disorders causing seizures (multiple sclerosis, diabetes melitus and systemic lupus erythematosus). Thus, while the invention relates to preventing the development of seizures in individuals suffering from such disorders or conditions, the invention does not provide a treatment for the underlying condition (i.e. stroke).

The term "brain injury likely to precipitate epilepsy or seizures, or cause or have caused brain damage" should be understood to mean stroke, trauma or other types of acute neurological injuries.

In the specification, the term "antagomir" should be understood to mean a novel class of chemically engineered oligonucleotides. Antagomirs are used to silence endogenous microRNA. An antagomir is a small synthetic oligonucleotide that is complementary to the specific miRNA target with either mispairing at the cleavage site of Arganoute 2 (Ago2) or some sort of base modification to inhibit Ago2 cleavage. Usually, antagomirs have some sort of modification, such as 2' methoxy groups, 3 '-cholesterol groups, phosphorothioates, to make it more resistant to degradation. It is believed that antagomirs inhibit by irreversibly binding the miRNA. An example of antagomirs suitable for use in inhibiting the activity of miR- 134 are molecules comprising the oligonucelotides CGTCTAGCCACCTAG (SEQ ID NO: 6) or CCTCTGGTCAACCAGTCAC (SEQ ID NO: 7), or variants thereof that have at least 80%, 85%, 90%, 95% or 99% sequence identity with SEQ ID NO: 6 or 7 when determined using the sequence alignment program BLAST, and which are capable of silencing human miR-134. Ideally, the antagomir molecule comprises a base modification to make it more resistance to degradation, for example a 2' methoxy group, 3 '-cholesterol group, or phosphorothioate, modification.

In this specification, the term "biological system" should be taken to include a cell, a cell line, a tissue sample, an organ, a unicellular or multicellular organism, or a lower or higher life form. Various delivery systems are known and can be used to administer a therapeutic of the invention, e.g., intra-nasally (see Figure 9). Methods of introduction include but are not limited to intradermal, intramuscular, intraperitoneal, intravenous, subcutaneous, intranasal, epidural, intranasal, intracerebral, and oral routes. The compositions may be administered by any convenient route, for example by infusion or bolus injection, by absorption through epithelial or mucocutaneous linings (e.g. , oral mucosa, rectal and intestinal mucosa, etc.) and may be administered together with other biologically active agents. Administration can be systemic or local. In addition, it may be desirable to introduce the compositions of the invention into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent. Preferably, the therapeutic is delivered to the CNS or PNS. Delivery means include intravenous delivery, oral delivery, intramuscular delivery, intrathecal delivery, and inhaled delivery. Methods for achieving these means of delivery will be well known to those skilled in the art of drug delivery, and include: •Delivered intrathecially by mini-osmotic pump, (ref: Ignacio et al., Ann. N.Y. Acad. Sci. 2005, 1053: 121-136).
- Intramuscular delivery directly into muscle(s) by syringe or mini osmotic pump (Azzouz et al., Nat Med. 2005;11(4):429-33). •Intraperitoneal- for systemic administration - directly administered to peritoneum by syringe or mini osmotic pump (Kieran et al., Nat Med 2004; 10(4):402).
- Subcutaneous- for systemic administration - directly administered below the skin by syringe (Reinholz et al., Exp Neurol. 1999;159(1):204-16).
- Intraventricular- direct administration to the ventricles in the brain, by injection or using small catheter attached to an osmotic pump.(Sathasivam et al., 2005 Neuropath App Neurobiol; 31(5): 467)
- Implant- can be prepared in an implant (eg small silicon implant) that will release Active. Implant can be placed at muscles or directly onto the spinal cord (Kieran and Greensmith, 2004 Neurosci 125(2):427-39).

It may be desirable to administer the compositions of the invention locally to the area in need of treatment; this may be achieved, for example and not by way of limitation, by topical application, by injection, by means of a catheter, by means of a suppository, or by means of an implant, said implant being of a porous, non-porous, or gelatinous material, including membranes, such as sialastic membranes, or fibers.

The present invention also provides pharmaceutical compositions. Such compositions comprise a therapeutically effective amount of the therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. In this specification, the term "therapeutically effective amount" should be taken to mean an amount of therapeutic which results in a clinically significant inhibition, amelioration or reversal of development or occurrence of seizures or, in the case of treatment of stroke, clinically significant inhibition, amelioration or reversal of development of the effects of stroke.

The term "carrier" refers to a diluent, adjuvant, excipient, or vehicle with which the Therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the therapeutic, preferably in purified form, together with a suitable amount of carrier so as to provide the form for proper administration to the patient. The formulation should suit the mode of administration. In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to, ease pain at the, site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

Experiments detailed in this specification have been conducted on mice. However, miR-134 is highly conserved, between mouse, cyno and humans and mammals in general. Therefore the findings herein can reasonably expected to apply to mammals in general.

### DETAILED DESCRIPTION OF THE INVENTION

The present invention relates to pharmaceutical composition comprising an effective dosage of at least one anti miR-134 oligonucleotide for administration to a mammal suffering from a disease wherein lowering of the activity of miR-134 is beneficial, wherein the time interval between at least two successive administrations is at least 50 days.

In an embodiment, the at least two successive administrations can be single administrations. In this embodiment, a first, single administration is performed and is then followed by a second single administration which is performed at least 50 days later.

The inventors have found that the pharmaceutical comprising an effective dosage of at least one anti miR-134 oligonucleotide can be surprisingly dosed at a very low level (e.g. 0,1mg/kg or 0,1mg/kg) and can surprisingly be administered at very low frequency (only every 50 day or even more) while remaining efficacious. This is a major benefit for patients suffering from disease wherein lowering of the activity of miR-134 is beneficial, such as neurological disorders, for instance epilepsy. These and other surprising findings are described more in detail herein.

In some aspects, the at least two administrations are maintenance dosages of the antisense oligomer, such as a dosage which is sufficient to maintain an effective concentration of the oligomer in the subject, such as in a target tissue.

In an embodiment, the invention relates to a pharmaceutical composition comprising an effective dosage of at least one anti miR-134 oligonucleotide for administration to a mammal suffering from a disease wherein lowering of the activity of miR-134 is beneficial, wherein the time interval between each single administration of the pharmaceutical composition is at least 50 days.

In another embodiment, the invention relates to a pharmaceutical composition comprising an effective dosage of at least one anti miR-134 oligonucleotide for administration to a mammal suffering from a disease wherein lowering of the activity of miR-134 is beneficial, wherein the time interval between at least two successive administrations is at least 50 days.

In the embodiments of the invention herein, the time interval between administrations (single or successive) can be beyond 50 days. In an embodiment, it can be at least 70 days or at least 75, 80, 85, 90, 95, 100, 105, 110 or 120 or even beyond.

In an embodiment of the invention, the anti miR-134 oligonucleotide comprises 7 to 16 contiguous nucleotides complementary to sequence UCUCUCCCUCUGGUCAACCAGUCACAAGGCU or comprises 7 to 22 contiguous nucleotides complementary to the hsa-miR-134 (UGUGACUGGUUGACCAGAGGGG, SEQ ID NO 1).

In an embodiment of the invention, the anti miR-134 oligonucleotide is selected from the group consisting of the following sequences: CCCCTCTGGTCAACCAGTCACA, CGTCTAGCCACCTAG and CCTCTGGTCAACCAGTCAC or variants thereof that have at least 80%, 85%, 90%, 95% or 99% sequence identity therewith.

In an embodiment of the invention, the anti miR-134 oligonucleotide is incapable of recruiting RNaseH.

In an embodiment of the invention, the anti miR-134 oligonucleotide, contains at least one LNA nucleotide.

In an embodiment of the invention the anti miR-134 oligonucleotide is fully phosphorothioate.

In an embodiment of the invention the anti miR-134 oligonucleotide is a mixmer. The mixmer length can be 10 to 23 nucleotides, although the person skilled in the art will appreciate that it can be a different length as described herein and in the literature. The anti miR-134 oligonucleotide can also be a totalmer. Such totalmer's length can be for example 7 to 10 nucleotides, although the person skilled in the art will appreciate that it can be a different length as described herein and in the literature.

In an embodiment of the invention the anti miR-134 oligonucleotide contains at least one phosphorothioate and at least one LNA is present and C is 5 methylC.

In an embodiment of the invention anti miR-134 oligonucleotide is 5'-TgGtcAAccAgTcAC-3', wherein Capital letters indicate beta-D-oxy LNA, lower case DNA and LNA C is 5 methylC and wherein it is a fully phosphorothioate oligonucleotide. In the experiments described herein this anti miR-134 is referred to as compound A.

In an embodiment of the invention the anti miR-134 oligonucleotide can be administered at about 0.05 mg/kg to about 0.15 mg/kg. In an embodiment of the invention the anti miR-134 oligonucleotide can be administered at about 0.1 mg/kg. In an embodiment of the invention the anti miR-134 oligonucleotide can be administered at about 0.2 mg/kg.

In an embodiment of the invention, the oligonucleotides useful according to the invention can contain at least one of the nucleotide analogues can be chosen from the group consisting of: 2'-O-alkyl-RNA monomers, beta-D-oxy-LNA, 6'methyl beta-D-oxy LNA and ENA. In an embodiment of the invention the nucleotide analogues are locked nucleic acid (LNA).

In another aspect, the invention relates to a method of treatment of a mammal suffering from pre-existing epilepsy comprising administering a pharmaceutical composition according to the invention. In an embodiment, the pharmaceutical composition is administered as a single injection and wherein seizure suppression reaches at least 50% and lasts for at least 50 days after injection. In an embodiment, the pharmaceutical composition is administered as a single injection and wherein seizure suppression reaches at least 60% or 70% or 80% or 90% and lasts for at least 50 days after injection. In certain embodiments time interval between administrations (single or successive) can be beyond 50 days. In an embodiment, seizure suppression can last at least 70 days or at least 75, 80, 85, 90, 95, 100, 105, 110 or 120 or even beyond.

In an embodiment, the pharmaceutical composition is administered via intracerebroventricular (ICV). In an embodiment, the pharmaceutical composition is administered via intracerebroventricular (ICV) as a single injection. In an embodiment, the pharmaceutical composition is administered at least twice via intracerebroventricular (ICV) as single injections.

In another aspect, the invention relates to an anti miR-134 oligonucleotide for use in the treatment of a neurological disorder, such as a brain-related disorder characterized by development of seizures, wherein the time interval between two successive administrations is at least 50 days. In an embodiment, seizure suppression can last at least 70 days or at least 75, 80, 85, 90, 95, 100, 105, 110 or 120 or even beyond.

In another aspect, the invention relates to the use of an anti-miR-134 oligonucleotide in the manufacture of a medicament for the treatment of neurological disorder, such as a brain-related disorder characterized by development of seizures, wherein the medicament is for multiple administration to a subject wherein the time interval between two successive administrations is at least about 50 days. In an embodiment, seizure suppression can last at least 70 days or at least 75, 80, 85, 90, 95, 100, 105, 110 or 120 or even beyond. The anti miR-134 oligonucleotide that can be used in this aspect of the invention can be as defined herein.

The number of administrations may be one (single) or more than 2, such as 3, 4, 5, 6, 7, 8, 9, 10, 11, 12, 13, 14, 15, 16 or more treatments. As described herein, the actual number of administrations will depend on the nature of disease or disorder, for example. Diseases which may be cured will provide a definite end point to the administration regimen, whereas a disease or disorder may be treated over an extended period of time, effectively controlling symptoms, but may, in some embodiments not provide a cure. In such instances routine/regular administration may be continued for several months or years, until treatment is no longer desirable as determined by the medical practitioner. It will be noted that in some embodiments, administration regimens may be interrupted by a treatment pause, such a period of more than 125 days, or in some embodiments, a period of more than 2, 3, 5, or 6 months.

Further, in some embodiments, the invention relates to compositions comprising antisense oligomers that are essentially incapable of recruiting RNAseH, such as totalmers or mixmers, and wherein the compositions are made for maintaining a treatment of a patient, and wherein the dosages, such as the maintenance dosages, are provided with a long time interval in between each dosing.

The invention, in some aspects, provides for the use of an anti microRNA oligonucleotide in the preparation of a medicament for treating a disease or disorder in a mammal, wherein the disease or disorder is characterized by being sensitive to downregulation of a micro RNA,

The invention, in some aspects, provides for the use of an anti microRNA oligonucleotide in the preparation of a medicament for lowering of the activity of a particular micro RNA in a mammal, such as, but not limited to epilepsy.

(miRNAs) are ~22 nt endogenous non-coding RNAs that post-transcriptionally repress expression of protein-coding genes by base-pairing with the 3'-untranslated regions of the target mRNAs^{1,2,7}. Emerging evidence suggests that animal miRNAs play important roles in the control of many biological processes^{1-3,8}. In addition, miRNAs have been implicated in viral infections, cardiovascular disease, and neurological and muscular disorders, as well as in the onset and progression of cancers⁹⁻¹⁹.

The therapeutic value of antagonizing miR-134 is based on studies using animal modelling and vEEG recordings. In the following examples adult male C57BL/6 mice (~25 g) were used and obtained from RCSI's biomedical research facility (original stock from Harlan, Oxon, Bicester, U.K.).

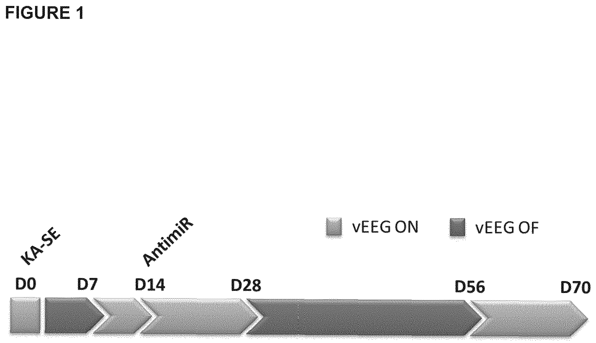

Referring to Figure 1, the animal modelling was as following: under vEEG monitoring, *status epilepticus* (SE) was triggered in freely-moving awake mice by intra-amygdala microinjection of KA (0.3 µg in 0.2µl) (Sigma-Aldrich, Ireland). After 40 min, all mice received lorazepam (8 mg/kg, i.p.) to curtail seizures and reduce morbidity and mortality. vEEG was recorded for 24 hr. to ensure all mice underwent similar SE, and telemetry units were turned off. Seven days later the EEG telemetry units were activated and continuous EEG recorded with video monitoring in the home cage to obtain an "epileptic baseline". On the 14^{th} day after *status epilepticus,* mice were injected with 0.5 nmol in 2 µl (about 0.1 mg/kg) of either compound: A, B or C via Intracerebroventricular (ICV) injection. Recordings were continued for the following two weeks ("activation period 1") and then interrupted for 4 weeks to conserve telemetry device battery life (battery lasts in total 6 weeks). On the 57^{th} day after *status epilepticus* recordings were re-activated for further two weeks ("activation period 2"). At the end of the entire experiment, mice were deeply anaesthetised and perfused through the heart with cold-buffered saline. Brains were removed for future histology analysis.

Epileptiform EEG during status epilepticus and spontaneous recurrent seizures were analysed using Neuroscore software (DSI). Data was quantified and Poisson regression and Zero-inflated Poisson regression were used to examine differences in the number of spontaneous seizures in the baseline and activation 1/2 periods, respectively. Deaths during the trial were analysed by Wilcoxon (Breslow) test. Significance was accepted at P < 0.05. Data are presented as mean ± standard error of the mean.

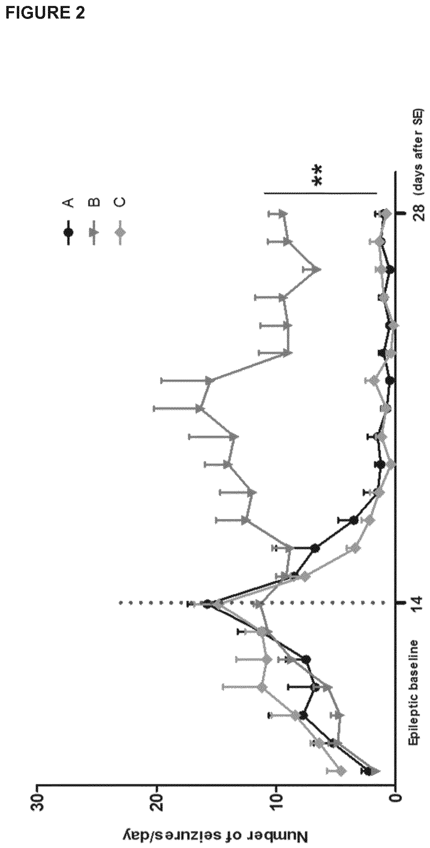

Day 14 was considered the injection day. Treatment phase (activation period 1) was defined as day 15 onward. Referring to Figure 2, mice with treated with 0.1 mg/kg compounds A and C showed 78% and 79% less spontaneous seizures, respectively, in comparison with group B (control) in the "activation period 1".

Figure 2 is a graph that shows the total number of seizures per day for experimental groups A (n=4), B (n=6) and C (n=5) for the "epileptic baseline" and "activation 1" periods. In this graph, P < 0.001.

The follow-up phase (activation period 2) was defined as day 57 onward. Both on-drug groups (A and C) had several seizure-free days (see raw data appendix). Again, the zero-inflated Poisson regression was used, and there is a significantly lower incidence of seizures in the drug treated animals (A and C) on follow-up (Drug A IRR 0·03, Drug C IRR 0·08, both P ≤ 0·001). Therefore, the conversion of IRR for "activation period 2" means that Group A presented 97% of reduction and Group C presented 92% of reduction in the number of spontaneous seizures in comparison with control (B) (see Figure 2). Again, the overall post hoc analysis showed that the effects of the drugs A and C do not differ significantly (Chi-squared = 0·44, P = 0·506). However, when the second analysis has been performed (using the ranking score system) a statistical difference between Groups A and C was verified in the activation 2 period (Group A treatment is slightly more potent than group C; P=0.044). Finally, as expected the differences between treatment groups versus control were ratified (Group A and C mice had 97% and 92% less spontaneous seizures, respectively, in comparison with group B).

Further, when mortality rate is analyzed over the whole period by Wilcoxon (Breslow) test comparing either A or C with group B, Groups A and C present lower mortality rate than Group B (borderline statistical significance: P=0·0516).

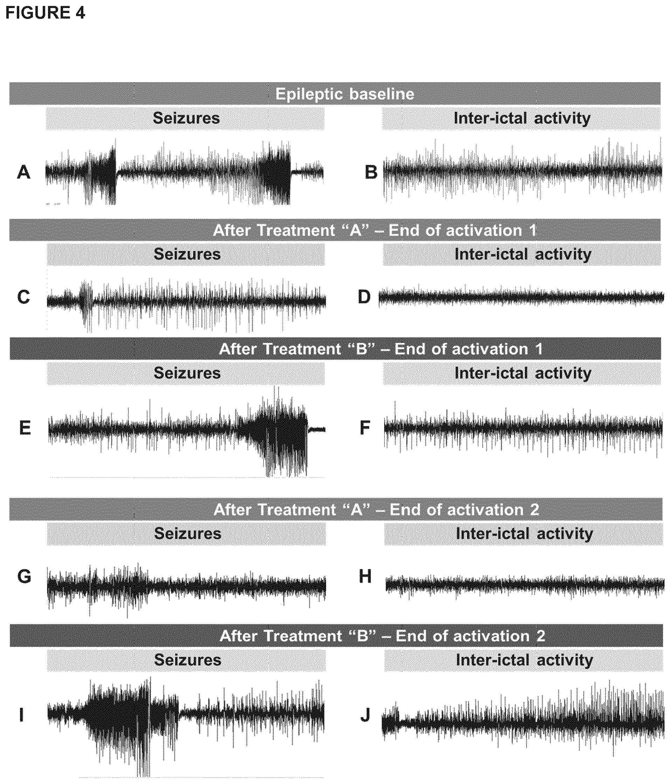

Figure 4 shows representative EEG recordings traces showing differences in seizure severity between Ant-134 (e.g. group A; grey tag) and control (red tag) groups. Figure (A-B) represents the occurrence of spontaneous seizures and the inter-ictal activity recording during a normal epileptic baseline period. Representative traces for "activation 1" (C-F) and "activation 2" (G-J) were obtained on the last day of recording of each respective period.

The inventors have found that anti miR-134 oligonucleotides, such as antagomirs targeting mir-134 are potently effective against spontaneous recurrent seizures in an adult mouse model of temporal lobe epilepsy. A a single ICV injection of compound A or C into mice with pre-existing spontaneous recurrent seizures resulted in a prompt, near-complete cessation of seizures. Spontaneous seizure rates were reduced not only during the initial activation period of two-weeks of recordings, starting two weeks after the epilepsy-triggering episode of status epilepticus, but were also evident during the two weeks of recordings starting 42 days after the treatment. Overall, the findings provide strong evidence that antagomirs targeting mir-134 produce potent and long-lasting suppression of spontaneous seizures when injected into already-epileptic mice.

There remains an urgent and unmet need for more effective therapies for the ~30% of patients with epilepsy who are refractory to available medication. Moreover, no existing treatment has been shown to improve the underlying pathophysiology. The present invention shows, for the first time that inhibiting mir-134 have effects on pre-existing epilepsy.

This target emerged from work showing this brain-enriched small noncoding RNA has important regulatory roles in neuronal microstructure and development and was consistently found to be up-regulated within epileptic foci in experimental and human epilepsy. Previous work showed that locked nucleic acid (LNA) antagomirs targeting miR-134 had promising anti-seizure effects. The experimental therapy was shown to suppress evoked seizures in various models when given as a pre-treatment and could suppress the later development of spontaneous seizures if given shortly after (e.g. 1 h) a status epilepticus. However, no previous work tested whether the treatment could prevent seizures once epilepsy was established. This is critical for clinical translation since there is currently no biomarker of epilepsy risk and therefore no obvious opportunity to apply an anti-epileptogenic treatment to patients that might develop epilepsy. Thus, the therapeutic market for epilepsy remains treating patients with pre-existing epilepsy.

The pharmaceutical composition of present invention shows can reduce or prevent spontaneous seizures in pre-existing epilepsy.

The model used is well established, featuring induction of status epilepticus by intra-amygdala KA that reliably leads to mice having recurrent spontaneous seizures within a few days. The epileptic seizures display clinically-relevant features such as tonic-clonic components and can be readily tracked using EEG and video monitoring. The model has been used to study a number of experimental therapies by the Henshall laboratory as well as other groups (Vezzani lab, McNamara lab). The spontaneous seizures in the model are resistant to some of the known anti-epileptic drugs and it is currently being trialed by the NIH's epilepsy therapy programme at the University of Utah (Wilcox lab). In the present study it was found that spontaneous recurrent seizures developed in all mice within a few days and continued at a rate of ~ 6 - 10 per day consistent with previous studies. All treatment groups showed a similar baseline rate consistent with the recorded status epilepticus being similar.

The main finding in the present invention was that ICV injection of compound A or C produced a prompt (within 24 h) reduction in spontaneous seizures relative to baseline seizure rates. Mice given compound B continued to experience frequent spontaneous seizures. During "activation period 1" the seizure frequency underwent a profound reduction in A and C group mice and remained lower through the two week recordings. Many mice experienced seizure-free days with either treatment. Given that tested anti-epileptic drugs such as carbamazepine are minimally effective against spontaneous seizures in this model, these results indicate, at a minimum, that antagomirs targeting mir-134 have potentially superior efficacy to prevent spontaneous seizures in temporal lobe epilepsy than existing treatments.

The pharmaceutical composition according to the invention shows a long effect as described herein.

The second, important finding according to the invention is that seizure rates in groups A and C were profoundly reduced during the second activation period that started 42 days after treatment.

In fact, seizure suppression relative to the other group was even greater during this activation period, with a >97% reduction in seizures in one of the groups. Together, this emonstrates that spontaneous recurrent seizures in already-epileptic mammals can be potently suppressed, indefinitely, by a single ICV injection of antagomirs targeting mir-134.

The inventors also noted that resting EEG was much improved in A and C group mice at the end of recordings compared to epileptic controls. These data support, again, a disease-modifying effect of the therapy to normalize brain excitability. Since epileptic activity is implicated in cognitive dysfunction, one may speculate that brain function may also be superior in A and C group mice. This was not tested presently due to adherence to the original study design and the practical difficulty in obtaining reliable behavioural data in cognitive tests where one group of animals are experiencing highly active epilepsy. Nevertheless, the antagomir treatment may confer additional benefits beyond simply reducing spontaneous seizures.

A major finding on the pharmaceutical composition of the invention is the exceptionally long duration of action of compounds A and C. Seizure suppression lasted over 50 days following a single injection.

It is tempting to speculate that this is evidence of a disease-modifying effect. That is, introduction of the antagomir to the epileptic brain not only suppressed ictogenesis but also altered the underlying pathophysiology giving rise to seizures. Indeed, acute anticonvulsant effects against status epilepticus using an Exiqon antagomir were found to last less than two weeks in the original studies by Jimenez-Mateos et al (2012). That is, if the antagomir was injected two weeks before KA-SE then seizure severity was normal at the time of triggering KA-SE. If the acute anticonvulsant effects of a single ICV injection of antagomir last only a couple of weeks then the reduced seizures observed during "activation period 1" could be a result of acute seizure-suppression (somewhat like a current AED works) but it is unlikely this could account for the reduced seizures during the second activation period. Thus, the simplest explanation of the results during the second activation period is that the injection of the antagomir had a disease-modifying effect, reducing the propensity of the epileptic focus to trigger seizures.

Sudden unexpected death (SUDEP) is a serious risk in patients with intractable seizures, particularly patients experiencing tonic-clonic seizures. In the present study occasional sudden deaths in mice that became epileptic was observed. These were rarely observed by a researcher (i.e. the mouse was found dead in the cage and review of the video showed in most cases that it occurred in the context of a recent seizure). This would be consistent with the clinical picture since the spontaneous seizures in this model are generalised and feature both tonic (e.g. Straub tail) and clonic (convulsions) components. The incidence of SUDEP in this model has not been systematically studied therefore there is no comparator/baseline data to compare whether any of the treatments increased or decreased the rate. Nevertheless, an additional finding of significance in the present study was reduced mortality rate in the mice given either compound A or C. This finding suggests the antagomir offers an additional clinical benefit relevant for some of the devastating paediatric epilepsies such as Dravet syndrome, which are associated with high rates of SUDEP. The mechanisms of SUDEP remain incompletely understood and therefore it is not known if the reduced mortality rate was a direct effect of the antagomir or secondary to an overall reduction in the occurrence of spontaneous seizures. Regardless, in addition to reducing spontaneous recurrent seizures, antagomirs targeting mir-134 may reduce SUDEP risk or occurrence.

Extracellular microRNAs could be potential biomarkers of epilepsy and serve as theranostic or prognostic markers of seizure risk or drug response.

We previously reported that patients with epilepsy had higher blood levels of mir134. It would be interesting to know if blood levels of mir-134 (or other, brain-expressed miRNAs) show proportional changes in levels in the seizure-suppressed mice compared to the animals with ongoing epileptic activity. However, blood in the mice was not sampled before, during or after treatment in the present studies due to the already complex and invasive procedures being applied. Nevertheless, future studies could attempt to combine the therapy with ongoing biofluid analysis to develop a combined therapeutic-diagnostic.

In summary, the present invention shows that that antagomirs targeting mir-134 potently suppress spontaneous recurrent seizures when injected into already-epileptic mice in a model of temporal lobe epilepsy. The seizure suppression reached as high as 97% and lasted at least 50 days after a single injection. These data strongly support further work on this molecule as a target for the treatment of refractory epilepsy.

### Length

In some embodiments the antisense oligonucleotide has a length of 7 - 25 (contiguous) nucleotides, such as 8, 9, 10, 11, 12, 13, 14, 15, 16, 17, 18, 19, 20, 21, 22, 23, or 24 (contiguous) nucleotides. In some embodiments, the antisense oligonucleotide has a length of 7 - 10 (contiguous) nucleotide, or in some instances 7 - 16 nucleotides. In some embodiments, the antisense oligonucleotide at least 8 (contiguous) nucleotides in length, between 10-17 or 10 - 16 or 10-15 (contiguous) nucleotides, such as between 12 - 15 (contiguous) nucleotides.

### Oligomers which are essentially incapable of recruiting RNAseH

EP 1 222 309 provides in vitro methods for determining RNaseH activity, which may be used to determine the ability to recruit RNaseH. A oligomer is deemed capable of recruiting RNase H if, when provided with the complementary RNA target, it has an initial rate, as measured in pmol/l/min, of at least 1 %, such as at least 5%, such as at least 10% or less than 20% of the equivalent DNA only oligonucleotide, with no 2' substitutions, with phosphorothioate linkage groups between all nucleotides in the oligonucleotide, using the methodology provided by Example 91 - 95 of EP 1 222 309.

In one embodimentIn some embodiments, an oligomer is deemed essentially incapable of recruiting RNaseH if, when provided with the complementary RNA target, and RNaseH, the RNaseH initial rate, as measured in pmol/l/min, is less than 1%, such as less than 5%,such as less than 10% or less than 20% of the initial rate determined using the equivalent DNA only oligonucleotide, with no 2' substitutions, with phosphorothioate linkage groups between all nucleotides in the oligonucleotide, using the methodology provided by Example 91 - 95 of EP 1 222 309.

It should be recognised that oligonucleotides which are mixmers or totalmers are usually essentially incapable of recruiting RNAseH and as such where the term is used essentially incapable or recruiting RNaseH herein, in some embodiments, such a term may be replaced with the term mixmer or totalmer, as defined herein, even if, in some instances such oligomers actually do possess significant ability to recruit RNaseH, such as when using DNA mixmers with alpha-L-oxy-LNA.

In some embodiments, the oligomer or contiguous nucleotide sequence thereof consists of a contiguous sequence of nucleotide analogues, such as affinity enhancing nucleotide analogues - referred to herein is as a 'totalmer'.

### Totalmers

A totalmer is a single stranded oligomer, or contiguous nucleotide sequence thereof, which does not comprise DNA or RNA nucleosides, and as such only comprises nucleoside analogue nucleosides. only comprises non-naturally occurring nucleotides.

The oligomer, or contiguous nucleotide sequence thereof, maybe a totalmer - indeed various totalmer designs are highly effective as therapeutic oligomers, particularly when targeting microRNA (antimiRs) or as splice switching oligomers (SSOs).

In some embodiments, the totalmer comprises or consists of at least one XYX or YXY sequence motif, such as a repeated sequence XYX or YXY, wherein X is LNA and Y is an alternative (i.e. non LNA) nucleotide analogue, such as a 2'-OMe RNA unit and 2'-fluoro DNA unit. The above sequence motif may, in some embodiments, be XXY, XYX, YXY or YYX for example.

In some embodiments, the totalmer may comprise or consist of a contiguous nucleotide sequence of between 8 and 16 nucleotides, such as 9, 10, 11, 12, 13, 14, or 15 nucleotides, such as between 8 and 12 nucleotides.

In some embodiments, the contiguous nucleotide sequence of the totalmer comprises of at least 30%, such as at least 40%, such as at least 50%, such as at least 60%, such as at least 70%, such as at least 80%, such as at least 90%, such as 95%, such as 100% LNA units. The remaining units may be selected from the non-LNA nucleotide analogues referred to herein in, such as those selected from the group consisting of 2'-O_alkyl-RNA unit, 2'-OMe-RNA unit, 2'-amino-DNA unit, 2'-fluoro-DNA unit, LNA unit, PNA unit, HNA unit, INA unit, and a 2'MOE RNA unit, or the group of 2'-OMe RNA unit and 2'-fluoro DNA unit.

In some embodiments the totalmer consist or comprises of a contiguous nucleotide sequence which consists only of LNA units.

In some embodiments, the totalmer may be targeted against a microRNA (i.e. be antimiRs) - as referred to in US provisional applications 60/979217 and 61/028062, and PCT/DK2008/000344, all of which are hereby incorporated by reference.

### Mixmers

The term 'mixmer' refers to oligomers, or contiguous nucleotide sequences thereof, which comprise DNA nucleosides and nucleoside analogue nucleosides, both naturally and non-naturally occurring nucleotides, where, as opposed to gapmers, tailmers, headmers and blockmers, there is no contiguous sequence of more than 5 naturally occurring DNA nucleotidesnucleosides , such as DNA units..

The oligomer, or contiguous nucleotide sequence thereof, according to the invention may be mixmers - indeed various mixmer designs are highly effective as therapeutic oligomers, particularly when targeting microRNA (antimiRs), microRNA binding sites on mRNAs (Blockmirs) or as splice switching oligomers (SSOs).

The oligomer may, , or contiguous nucleotide sequence thereof, in some embodiments, also be a mixmer and indeed, due to the ability of mixmers to effectively and specifically bind to their target, the use of mixmers as therapeutic oligomers are considered to be particularly effective in decreasing the target RNA.

In some embodiments, the mixmer comprises or consists of a contiguous nucleotide sequence of repeating pattern of nucleotide analogue and naturally occurring nucleotides , or one type of nucleotide analogue and a second type of nucleotide analogues. The repeating pattern, may, for instance be every second or every third nucleotide is a nucleotide analogue, such as LNA, and the remaining nucleotides are naturally occurring nucleotides, such as DNA, or are a 2'substituted nucleotide analogue such as 2'MOE of 2'fluoro analogues as referred to herein, or, in some embodiments selected form the groups of nucleotide analogues referred to herein. It is recognised that the repeating pattern of nucleotide analogues, such as LNA units, may be combined with nucleotide analogues at fixed positions - e.g. at the 5' or 3' termini.

In some embodiments the first nucleotide of the oligomer or mixmer, counting from the 3' end, is a nucleotide analogue, such as an LNA nucleotide.

In some embodiments, which maybe the same or different, the second nucleotide of the oligomer or mixmer, counting from the 3' end, is a nucleotide analogue, such as an LNA nucleotide.

In some embodiments, which maybe the same or different, the seventh and/or eighth nucleotide of the oligomer or mixmer, counting from the 3' end, are nucleotide analogues, such as LNA nucleotides.

In some embodiments, which maybe the same or different, the ninth and/or the tenth nucleotides of the oligomer or mixmer, counting from the 3' end, are nucleotide analogues, such as LNA nucleotides.

In some embodiments, which maybe the same or different, the 5' terminal of the foligomer or mixmer is a nucleotide analogue, such as an LNA nucleotide.

The above design features may, in some embodiments be incorporated into the mixmer design, such as antimiR mixmers.

In some embodiments, the mixmer does not comprise a region of more than 4 consecutive DNA nucleotide units or 3 consecutive DNA nucleotide units. In some embodiments, the mixmer does not comprise a region of more than 2 consecutive DNA nucleotide units. In some embodiments, the mixmer comprises at least a region consisting of at least two consecutive nucleotide analogue units, such as at least two consecutive LNA units.

In some embodiments, the mixmer comprises at least a region consisting of at least three consecutive nucleotide analogue units, such as at least three consecutive LNA units.

In some embodiments, the mixmer of the invention does not comprise a region of more than 7 consecutive nucleotide analogue units, such as LNA units. In some embodiments, the mixmer of the invention does not comprise a region of more than 6 consecutive nucleotide analogue units, such as LNA units. In some embodiments, the mixmer of the invention does not comprise a region of more than 5 consecutive nucleotide analogue units, such as LNA units. In some embodiments, the mixmer of the invention does not comprise a region of more than 4 consecutive nucleotide analogue units, such as LNA units. In some embodiments, the mixmer of the invention does not comprise a region of more than 3 consecutive nucleotide analogue units, such as LNA units. In some embodiments, the mixmer of the invention does not comprise a region of more than 2 consecutive nucleotide analogue units, such as LNA units.

In the mixmer embodiments, which refer to the modification of nucleotides in positions 3 to 8, counting from the 3' end, the LNA units may be replaced with other nucleotide anlogues, such as those referred to herein. "X" may, therefore be selected from the group consisting of 2'-O-alkyl-RNA unit, 2'-OMe-RNA unit, 2'-amino-DNA unit, 2'-fluoro-DNA unit, 2'-MOE-RNA unit, LNA unit, PNA unit, HNA unit, INA unit. "x" is preferably DNA or RNA, most preferably DNA.

In some embodiments, the mixmer, such as an antimiR mixmer, is modified in positions 3 to 8 *- i.e.* comprises at least one nucleotide analogue in positions 3 to 8, counting from the 3' end. The design of this sequence may be defined by the number of non-LNA units present or by the number of LNA units present. In some embodiments of the former, at least one, such as one, of the nucleotides in positions three to eight, counting from the 3' end, is a non-LNA unit. In some embodiments, at least two, such as two, of the nucleotides in positions three to eight, counting from the 3' end, are non-LNA units. In some embodiments, at least three, such as three, of the nucleotides in positions three to eight, counting from the 3' end, are non-LNA units. In some embodiments, at least four, such as four, of the nucleotides in positions three to eight, counting from the 3' end, are non-LNA units. In some embodiments, at least five, such as five, of the nucleotides in positions three to eight, counting from the 3' end, are non-LNA units. In some embodiments, all six nucleotides in positions three to eight, counting from the 3' end, are non-LNA units.

Alternatively defined, in some embodiments, the mixmer, such as an antimiR mixmer, according to the invention comprises at least one LNA unit in positions three to eight, counting from the 3' end. some embodiments, the mixmer, such as an antimiR mixmer, comprises one LNA unit in positions three to eight, counting from the 3' end. The substitution pattern for the nucleotides in positions three to eight, counting from the 3' end, may be selected from the group consisting of Xxxxxx, xXxxxx, xxXxxx, xxxXxx, xxxxXx and xxxxxX, wherein "X" denotes an LNA unit and "x" denotes a non-LNA unit.

In some embodiments, the mixmer, such as an antimiR mixmer, comprises at least two LNA units in positions three to eight, counting from the 3' end. In some embodiments thereof, the mixmer comprises two LNA units in positions three to eight, counting from the 3' end. The substitution pattern for the nucleotides in positions three to eight, counting from the 3' end, may be selected from the group consisting of XXxxxx, XxXxxx, XxxXxx, XxxxXx, XxxxxX, xXXxxx, xXxXxx, xXxXxx, xXxXxx, xxXXxx, xxXxXx, xxXxxX, xxxXXx, xxxXxX and xxxxXX, wherein "X" denotes an LNA unit and "x" denotes a non-LNA unit. In an embodiment, the substitution pattern for the nucleotides in positions three to eight, counting from the 3' end, is selected from the group consisting of XxXxxx, XxxXxx, XxxxXx, XxxxxX, xXxXxx, xXxxXx, xXxxxX, xxXxXx, xxXxxX and xxxXxX, wherein "X" denotes an LNA unit and "x" denotes a non-LNA unit. In some embodiments, the substitution pattern for the nucleotides in positions three to eight, counting from the 3' end, is selected from the group consisting of xXxXxx, xXxxXx, xXxxxX, xxXxXx, xxXxxX and xxxXxX, wherein "X" denotes an LNA unit and "x" denotes a non-LNA unit. In some embodiments, the substitution pattern for the nucleotides in positions three to eight, counting from the 3' end, is selected from the group consisting of xXxXxx, xXxxXx and xxXxXx, wherein "X" denotes an LNA unit and "x" denotes a non-LNA unit. In some embodiments, the substitution pattern for the nucleotides in positions three to eight, counting from the 3' end, is xXxXxx, wherein "X" denotes an LNA unit and "x" denotes a non-LNA unit.

In some embodiments, the mixmer, such as an antimiR mixmer, comprises at least three LNA units in positions three to eight, counting from the 3' end. In an embodiment thereof, the mixmer comprises three LNA units in positions three to eight, counting from the 3' end. The substitution pattern for the nucleotides in positions three to eight, counting from the 3' end, may be selected from the group consisting of XXXxxx, xXXXxx, xxXXXx, xxxXXX, XXxXxx, XXxxXx, XXxxxX, xXXxXx, xXXxxX, xxXXxX, xXxXxx, XxxXXx, XxxxXX, xXxXXx, xXxxXX, xxXxXX, xXxXxX and XxXxXx, wherein "X" denotes an LNA unit and "x" denotes a non-LNA unit. In some embodiments, the substitution pattern for the nucleotides in positions three to eight, counting from the 3' end, is selected from the group consisting of XXxXxx, XXxxXx, XXxxxX, xXXxXx, xXXxxX, xxXXxX, XxXXxx, XxxXXx, XxxxXX, xXxXXx, xXxxXX, xxXxXX, xXxXxX and XxXxXx, wherein "X" denotes an LNA unit and "x" denotes a non-LNA unit. In some embodiments, the substitution pattern for the nucleotides in positions three to eight, counting from the 3' end, is selected from the group consisting of xXXxXx, xXXxxX, xxXXxX, xXxXXx, xXxxXX, xxXxXX and xXxXxX, wherein "X" denotes an LNA unit and "x" denotes a non-LNA unit. In some embodiments, the substitution pattern for the nucleotides in positions three to eight, counting from the 3' end, is xXxXxX or XxXxXx, wherein "X" denotes an LNA unit and "x" denotes a non-LNA unit. In some embodiments, the substitution pattern for the nucleotides in positions three to eight, counting from the 3' end, is xXxXxX, wherein "X" denotes an LNA unit and "x" denotes a non-LNA unit.

In some embodiments, the mixmer comprises at least four LNA units in positions three to eight, counting from the 3' end. In some embodiments thereof, the mixmer comprises four LNA units in positions three to eight, counting from the 3' end. The substitution pattern for the nucleotides in positions three to eight, counting from the 3' end, may be selected from the group consisting of xxXXXX, xXxXXX, xXXxXX, xXXXxX, xXXXXx, XxxXXX, XxXxXX, XxXXxX, XxXXXx, XXxxXX, XXxXxX, XXxXXx, XXXxxX, XXXxXx and XXXXxx, wherein "X" denotes an LNA unit and "x" denotes a non-LNA unit.

In some embodiments, the mixmer according to the present invention comprises at least five LNA units in positions three to eight, counting from the 3' end. In some embodiments thereof, the mixmercomprises five LNA units in positions three to eight, counting from the 3' end. The substitution pattern for the nucleotides in positions three to eight, counting from the 3' end, may be selected from the group consisting of xXXXXX, XxXXXX, XXxXXX, XXXxXX, XXXXxX and XXXXXx, wherein "X" denotes an LNA unit and "x" denotes a non-LNA unit.

In some embodiments, said non-LNA unit is another nucleotide analogue unit.

In some mixmer embodiments the substitution pattern for the nucleotides from position 11, counting from the 3' end, to the 5' end may include nucleotide analogue units (such as LNA) or it may not. In some embodiments, the mixmer comprises at least one nucleotide analogue unit (such as LNA), such as one nucleotide analogue unit, from position 11, counting from the 3' end, to the 5' end. In some embodiments, the mixmer comprises at least two nucleotide analogue units, such as LNA units, such as two nucleotide analogue units, from position 11, counting from the 3' end, to the 5' end.

In some embodiments which refer to the modification of nucleotides in the nucleotides from position 11 to the 5' end of the oligomer, the LNA units may be replaced with other nucleotide anlogues, such as those referred to herein. "X" may, therefore be selected from the group consisting of 2'-O-alkyl-RNA unit, 2'-OMe-RNA unit, 2'-amino-DNA unit, 2'-fluoro-DNA unit, LNA unit, PNA unit, HNA unit, INA unit. "x" is preferably DNA or RNA, most preferably DNA.

In some embodiments, the mixmer has the following substitution pattern, which is repeated from nucleotide eleven, counting from the 3' end, to the 5' end: xXxX or XxXx, wherein "X" denotes an LNA unit and "x" denotes a non-LNA unit. In another embodiment, the mixmer has the following substitution pattern, which is repeated from nucleotide eleven, counting from the 3' end, to the 5' end: XXxXxx, XXxxXx or XxXxxX, wherein "X" denotes an LNA unit and "x" denotes a non-LNA unit. In yet another embodiment, the mixmer has the following substitution pattern, which is repeated from nucleotide eleven, counting from the 3' end, to the 5' end: XXXxXXXx, XXxXxXxX, XXXxxxXX or XXxXxxXX, wherein "X" denotes an LNA unit and "x" denotes a non-LNA unit.

The specific substitution pattern for the nucleotides from position 11, counting from the 3' end, to the 5' end depends on the number of nucleotides in the mixmer. In a preferred embodiment, the mixmer contains 12 nucleotides and the substitution pattern for positions 11 to 12, counting from the 3' end, is selected from the group consisting of xX and Xx, wherein "X" denotes an LNA unit and "x" denotes a non-LNA unit. In some embodiments, the substitution pattern for positions 11 to 12, counting from the 3' end, is xX, wherein "X" denotes an LNA unit and "x" denotes a non-LNA unit. Alternatively, no LNA units are present in positions 11 to 12, counting from the 3' end, i.e. the substitution pattern is xx.

In some embodiments, the mixmer contains 12 nucleotides and the substitution pattern for positions 10 to 12, counting from the 3' end, is selected from the group consisting of Xxx, xXx, xxX, XXx, XxX, xXX and XXX, wherein "X" denotes an LNA unit and "x" denotes a non-LNA unit. In some embodiments thereof, the substitution pattern for positions 10 to 12, counting from the 3' end, is selected from the group consisting of xXx, xxX and xXX, wherein "X" denotes an LNA unit and "x" denotes a non-LNA unit. In some embodiments, the substitution pattern for positions 10 to 12, counting from the 3' end, is xxX, wherein "X" denotes an LNA unit and "x" denotes a non-LNA unit. Alternatively, no LNA units are present in positions 10 to 12, counting from the 3' end, *i.e.* the substitution pattern is xxx.

In some embodiments, the mixmer contains an LNA unit at the 5' end. In some embodiments, the mixmer contains an LNA unit at the first two positions, counting from the 5' end. The mixmer may also contain one or more of the structural features which are specified in the context of the antimiR herein - either the context thatthe mixmer contains a similar pattern and number of nucleotides/nucleotide analogues (e.g. X and x or X and Y).

The oligomer may, in some embodiments, be either i) fully complementary to a sub-sequence of contiguous nucleotides present in theRNA target, or ii) comprises no more than a single mismatch with the complement of a sub-sequence of contiguous nucleotides present in said RNA target. As such the oligonucleotide is an antisense oligonucleoitde - in that it is either fully complementary to the (corresponding) target sequence, or comprises no more than a single mismatch with the target sequence. The RNA target is typically associated with a medical condition or disease, and may in some embodiments, be a microRNA or a mRNA, for example. The oligomer may therefore be, for example, an antimiR, a microRNA mimic, a microRNA blockmir, or an antisense oligomer.

The oligomer may therefore be an antimir which targets (i.e. comprises or consists of a contiguous nucleotide sequence which is fully complementary to (a corresponding region of) one of the microRNAs listed above or comprises of no more than a single mismatch thereto. Such oligonucleotides may be referred to as anti-microRNA oligonucleotides.

A particular microRNA may for example be any of the individual microRNAs disclosed to or referred to herein, including all microRNAs published in miRBase and the patent applications referred to here. The term particular in this context may refer to a microRNA.

### Modulators of microRNA's useful in the invention

Compounds for use in the present invention are pharmaceutically active mir-134 antagomirs. In the context of this invention pharmaceutically active compounds denotes compounds that may have the potential, based on pre-clinical data such as in vitro (e.g. cell based assays) or in vivo (e.g. animal data such as mouse data), to provide a benefit to a subject. Such subject can be a mammal such as a mouse or a human.

Pharmaceutically active mir-134 antagomirs can be found in the microRNA database "mirbase" (http://microrna.sanger.ac.uk/sequences/). Pharmaceutically active mir-134 antagomirs that are described in patent application WO 2013/045652 to the Royal College of Surgeons in Ireland can be used in the compositions administered according to the present invention.

In one preferred embodiment, the modulator comprises an antisense LNA oligonucleotide. In one specially preferred embodiment, the modulator comprises an oligonucleotide which is between 7 and 25 nucleotides long and comprises at least one LNA. In some embodiments, the microRNA modulator comprises an oligonucleotide which is between 7 and 25 nucleotides long and comprises at least one LNA, and further comprises at least one other affinity increasing nucleotide analogue. In some embodiments, the oligonucleotide of the invention comprise phosphorothioate linkages.

In some embodiments the anti-miR-134 oligonucleotide is a LNA antimiR (see WO2007/112754 for example). LNA antimiRs are antisense oligonucleotides complementary to the mature microRNA target which comprise LNA nucleosides, and may further comprise phosphorothioate internucleotide linkages. LNA antimiRs may further comprise other nucleotides such as DNA and/or 2'-O-methoxyethyl (MOE) nucleosides (See EP1931780 for example). It is advantageous for targeting mature microRNAs that anti-miR-134 oligonucleotides do not recruit RNaseH1, and as such it is advantageous that the anti-miR-134 oligonucleotide, such as the LNA antimiRs do not contain regions of 4 or more contiguous DNA nucleosides.

Anti-microRNA LNA phosphorothioates may be ordered from Qiagen (https://www.qiagen.com/dk/shop/pcr/primer-sets/mircury-lna-mirna-inhibitors/#orderinginformation). Compound C (Exiqon antogomir) used in the experiments described herein can be obtained from this source.

Advantageously anti-miR-134 oligonucleotides which target the mature microRNA-134 target nucleic acid comprises the complement to the microRNA-134 seed region (nucleotides 2 - 7 from the 5' end of hsa-miR-134). In some embodiments the anti-miR 134 oligonucleotide comprises at least 1, such as at least 2 or at least 3 LNA nucleotides which are located in positions which are complementary to the microRNA-134 seed region (e.g the anti miR-134 oligonucleotide comprises the sequence CAGTCAC or AGTCAC).

LNA antimiRs may be fully phosphorothioate or may be partially phosphorothioate. For systemic administration a high proportion of fully phosphorothioate antisesen oligonucleotides may be preferred, however for local administration to the CNS, it may be desirable to use partial phosphorothioates.

In some embodiments the anti-miR-134 oligonucleotide is 7 - 10 contiguous LNA nucleotides in length (also refered to as Tiny LNAs, see Obad et al., Nat Genet. 2011 Mar 20;43(4):371-8, and WO2009043353 and comprises the complement of the microRNA-134 seed region. Examples of TINY LNA inhibitors of hsa-miR-134 include: ACCAGTCAC, CCAGTCAC & CAGTCAC, wherein each nucleotide is a LNA nucleoside such as beta-D-oxy LNA, and all internucleoside linkages are phosphorothioate. Use of partial phosphorothioates may also be used, for example the internucleoside linkages between the terminal nucleotises may be phosphorothioate, and the remaining internucleoside linkages may be phosphodiester.

In an embodiment, the pharmaceutical composition comprise an anti-miR-134 oligomer having the sequence: 5'-TgGtcAAccAgTcAC-3' (SEQ ID NO:8) also referred to as compound A herein, wherein Capital letters indicate beta-D-oxy LNA, lower case DNA and LNA C is 5 methylC and wherein such compound as a fully phosphorothioate backbone. This oligomer can be prepared as described in WO 2007/112754. Other oligomers of WO 2007/112754 can be used according to the invention, the content of which is hereby incorporated by reference.

In a preferred embodiment, the microRNA modulater is an LNA antisense oligomer comprising or consisting of any one of the sequences listed in Table 1.

**Table 1.** The following specific sequences or compounds, which may be used in the methods of the present invention, such as in the treatment of a disease, such as a disease where expression/over-expression of miR-134 are indicated such as those diseases illustrated in table 1. The compounds are preferably fully phosphorothioate and each nucleotide is a LNA nucleotide, such as beta-D-oxy LNA. LNA cytosine may be 5'methyl cytosine. Equivalent antimiRs can be designed by matching the -2 to -8/-9 or -10 positions (for 7, 8 or 9mers) of mature microRNAs (counting from the terminal 5' nucleotide of the microRNA (i.e. at the -1 position).

**Table 1**

| **Sequence** | **SEQ ID** |
|---|---|
| CCCCTCTGGTCAACCAGTCACA | 5 |
| CGTCTAGCCACCTAG | 6 |
| CCTCTGGTCAACCAGTCAC | 7 |
| 5'-TgGtcAAccAgTcAC-3'* | 8 |

| | |
|---|---|
| *wherein Capital letters indicate beta-D-oxy LNA, lower case DNA and LNA C is 5 methylC and wherein such compound as a fully phosphorothioate backbone. | |

Numerous other chemistries and designs of anti-microRNA oligonucleotides are known in the art.

For microRNA inhibition in vivo numerous chemistries and designs have been used in the art, including non RNaseH recruiting antisense oligonucleotide targeting mature microRNA targets, e.g.:
Full 2'-O-methoxyethyl phosphorothioates as reported in WO2005013901, Esau et al., Cell Metab. 2006 Feb;3(2):87-98 and Davis et al, Nucleic Acids Res. 2006 May 11;34(8):2294-304.
2'-O-methoxyethyl / 2' fluoro mixmer phosphorothioates - See Davis et al., Nucleic Acids Res. 2009 Jan;37(1):70-7.
2'-O-methyl AntagomiRs - fully 2'-O-methyl modified full complements of mature microRNAs, where the 5'and 3' regions are phosphorothioate with an internal region of phosphodiester, incorporating a cholesterol conjugate (Krutzfeldt et al., Nature. 2005 Dec 1;438(7068):685-9)

For targeting pre-microRNA targets, RNaseH recruiting designs are reported to be functional, see WO2005013901 which discloses 2'-O-methoxyethy gapmers targeting pre-miRNA target sequences.

### Pharmaceutical compositions and methods of treatment

The antisense oligonucleotide or conjugate or pharmaceutical composition thereof, is typically administered to the subject in an effective dose - which may for example be determined by a dose which is sufficient to down-regulate the target RNA, or activity thereof, to a significant level over the time period between successive administration dosages, such as a level which is a therapeutic benefit to the subject. In some embodiments, the target RNA, or activity thereof is down-regulated by at least 10%, such as at least 20%, at least 30%, at least 40%, at least 50%, at least 60%, at least 70% or at least 80% or at least 90% during the time period between successive administration dosages. The pharmaceutical compositions of the invention may in some embodiments be made for administration to provide for an initial dosage build up phase, which may, depending on the disease pathology, be followed by a maintenance dosage scheme for the purpose of maintaining a concentration of the compound in the subject, such as in a target tissue of the subject, which will be effective in the treatment of the disease. The effectiveness of the dosages may in example be measured by observation of a disease parameter indicative of the state of the disease, or may depending on the target tissue, be measurable by observation of various tissue parameters, such as activity of the target RNA or amount of viral genome, or in alternative example on a measurable disease state dependent parameter in plasma. However, in some diseases, in non limiting example such a disease could be a viral disease, after the build up phase, a maintenance dosage could be given for a time period wherein the purpose is to maintain a relatively high activity or concentration of the compound in the target tissue, while e.g. the viral titre is decreased or other disease parameters are improved, after which the interval between each dosing could be increased or the dosage given at each dosing could be decreased or both, in order to maintain the disease at the new low level using the minimal needed effective dosage and at the same time obtain minimum side effects and the least inconvenience for the patient by having a high time interval in between administrations.

In some embodiments, after the build up phase, a maintenance dosage will be administered wherein the purpose is to maintain an effective concentration in the target tissue, in order to obtain the desired effect on important disease parameters, wherein the time interval in between each administration is large to avoid the inconvenience for the patient of the administration, and the dosage is kept to a minimum to avoid side effects while still maintaining the effect on the selected disease parameters.

In some embodiments, the time interval between the at least two dosages, such as maintenance dosages, is selected from any one of at least 14 days, such as at least 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124 or at least 125 days. In some embodiments, the time interval between said at least two dosages, such as maintenance dosages, is selected from any one of at least 2 weeks, such as at least 3, 4, 5, 6, 7, 8, 9, 10, 11, 12, 13, 14, 15, 16, 17 or at least 18 weeks. In some embodiments, the time interval between said at least two dosages, such as maintenance dosages, is selected from any one of at least ½ month, such as at least 1, 1 ½, 2, 2 ½, 3, 3 ½, 4 or at least 4 ½ month.

In some embodiments the time interval between said at least two dosages is up to six months, or in some embodiments is less than 6 months.

In some embodiments, the treatment will be maintained for as long as the patient has symptoms of active disease. In some embodiments, the treatment may be paused for a period, and subsequently resumed by an initial period of high or frequent dosing to re-build effective tissue concentrations of the compound, followed by maintenance treatment according to the description.

In one preferred embodiment, the time interval between the at least two dosages, such as the maintenance dosages, is at least 14 days. In one preferred embodiment, the time interval between dosages is at least 21 days. In one preferred embodiment, the time interval between dosages is at least 4 weeks. In one preferred embodiment, the time interval between dosages is at least 5 weeks. In one preferred embodiment, the time interval between dosages is at least 6 weeks. In one preferred embodiment, the time interval between dosages is at least 7 weeks. In one preferred embodiment, the time interval between dosages is at least 8 weeks. Such dosages may be maintenance dosages.

In some embodiments, the dosage of the compound administered at each dosing, such as unit dose, is within the range of 0.01 mg/kg - 25 mg/kg. In some embodiments, the dosage, such as unit dose, of the compound administered at each dosing is within the range of 0.05 mg/kg - 20 mg/kg. In some embodiments, the dosage (such as unit dose) of the compound administered at each dosing is within the range of 0.1 mg/kg - 15 mg/kg. In some embodiments, the (such as unit dose) dosage of compound administered at each dosing is within the range of 1 mg/kg - 15 mg/kg. In some embodiments, the dosage of the compound administered at each dosing is within the range of 1 mg/kg - 10 mg/kg. In some embodiments, the dosage (such as unit dose) of the compound administered at each dosing is within the range of 0.01 mg/kg - 25 mg/kg, such as about 0.01, 0.05, 0.1, 0.2, 0.3, 0.4, 0.5, 0.6, 0.7, 0.8, 0.9, 1, 1.25, 1.5, 1.75, 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.25, 7.5, 7.75, 8, 8.25, 8.5, 8.75, 9, 9.25, 9.5, 9.75, 10, 10.25, 10.5, 10.75, 11, 11.25, 11.5, 11.75, 12, 12.25, 12.5, 12.75, 13, 13.25, 13.5, 13.75, 14, 14.25, 14.5, 14.75, 15, 16, 17, 18, 19, 20, 21, 22, 23, 24 or such as about 25 mg/kg, each of which are individual embodiments.

In some embodiments, the compositions of the invention (such as unit dose) are made for parenteral administration methods, such as in non limiting example, intra venous, sub cutaneous, intra peritoneal, intra cerebro vascular, intra nasal. In some embodiments, the administration is oral.

The oligomer of the invention may be used in pharmaceutical formulations and compositions. Suitably, such compositions comprise a pharmaceutically acceptable diluent, carrier, salt or adjuvant. PCT/DK2006/000512 provides suitable and preferred pharmaceutically acceptable diluent, carrier and adjuvants - which are hereby incorporated by reference. Suitable dosages, formulations, administration routes, compositions, dosage forms, combinations with other therapeutic agents, pro-drug formulations are also provided in PCT/DK2006/000512 - which are also hereby incorporated by reference.
Preferably the pharmaceutical composition of the invention further comprises a pharmaceutically acceptable carrier.

Preferably, the compound of the invention is included in a unit formulation (i.e. unit dose) such as in a pharmaceutically acceptable carrier or diluent in an amount sufficient to deliver to a patient a therapeutically effective amount without causing serious side effects in the treated patient.. However, in some forms of therapy, serious side effects may be acceptable in terms of ensuring a positive outcome to the therapeutic treatment.

The dosage of the pharmaceutical composition is dependent on severity and responsiveness of the disease state to be treated, and the course of treatment lasting from several days to several months, or until a cure is effected or a diminution of the disease state is achieved. Optimal dosing schedules can be calculated from measurements of drug accumulation in the body of the patient. Optimum dosages may vary depending on the relative potency of individual oligonucleotides. Generally it can be estimated based on EC₅₀s found to be effective in *in vitro* and *in vivo* animal models. In general, dosage is from 0.01 µg to 1 g per kg of body weight, and may be given once or more daily, weekly, monthly or yearly, or even once every 2 to 10 years or by continuous infusion for hours up to several months. The repetition rates for dosing can be estimated based on measured residence times and concentrations of the drug in bodily fluids or tissues. Following successful treatment, it may be desirable to have the patient undergo maintenance therapy to prevent the recurrence of the disease state.

The formulated drug may comprise pharmaceutically acceptable binding agents and adjuvants. Capsules, tablets and pills etc. may contain for example the following compounds: microcrystalline cellulose, gum or gelatin as binders; starch or lactose as excipients; stearates as lubricants; various sweetening or flavouring agents. For capsules the dosage unit may contain a liquid carrier like fatty oils. Likewise coatings of sugar or enteric agents may be part of the dosage unit. The oligonucleotide formulations may also be emulsions of the active pharmaceutical ingredients and a lipid forming a micellular emulsion.

The pharmaceutical compositions of the present invention may be administered in a number of ways depending upon whether local or systemic treatment is desired and upon the area to be treated. Administration may be (a) oral (b) pulmonary, e.g., by inhalation or insufflation of powders or aerosols, including by nebulizer; intratracheal, intranasal, (c) topical including epidermal, transdermal, ophthalmic and to mucous membranes including vaginal and rectal delivery; or (d) parenteral including intravenous, intraarterial, subcutaneous, intraperitoneal or intramuscular injection or infusion; or intracranial, e.g., intrathecal or intraventricular, administration. In some embodiments the active oligo is administered IV, IP, orally, topically or as a bolus injection or administered directly in to the target organ. In an exemplary embodiment, each dosage is administered in via parenteral injection or infusion, including intravenous, intraarterial, subcutaneous, intraperitoneal or intramuscular injection or infusion; or intracranial, e.g., intrathecal or intraventricular, administration.

Pharmaceutical compositions and formulations for topical administration may include transdermal patches, ointments, lotions, creams, gels, drops, sprays, suppositories, liquids and powders. Conventional pharmaceutical carriers, aqueous, powder or oily bases, thickeners and the like may be necessary or desirable. Preferred topical formulations include those in which the oligonucleotides of the invention are in admixture with a topical delivery agent such as lipids, liposomes, fatty acids, fatty acid esters, steroids, chelating agents and surfactants. Compositions and formulations for oral administration include but is not restricted to powders or granules, microparticulates, nanoparticulates, suspensions or solutions in water or non-aqueous media, capsules, gel capsules, sachets, tablets or minitablets. Compositions and formulations for parenteral, intrathecal or intraventricular administration may include sterile aqueous solutions which may also contain buffers, diluents and other suitable additives such as, but not limited to, penetration enhancers, carrier compounds and other pharmaceutically acceptable carriers or excipients.

Pharmaceutical compositions of the present invention include, but are not limited to, solutions, emulsions, and liposome-containing formulations. These compositions may be generated from a variety of components that include, but are not limited to, preformed liquids, self- emulsifying solids and self-emulsifying semisolids. Delivery of drug to tumour tissue may be enhanced by carrier-mediated delivery including, but not limited to, cationic liposomes, cyclodextrins, porphyrin derivatives, branched chain dendrimers, polyethylenimine polymers, nanoparticles and microspheres (Dass CR. J Pharm Pharmacol 2002; 54(1):3-27).

The pharmaceutical formulations of the present invention, which may conveniently be presented in unit dosage form, may be prepared according to conventional techniques well known in the pharmaceutical industry. Such techniques include the step of bringing into association the active ingredients with the pharmaceutical carrier(s) or excipient(s). In general the formulations are prepared by uniformly and intimately bringing into association the active ingredients with liquid carriers or finely divided solid carriers or both, and then, if necessary, shaping the product.

The compositions of the present invention may be formulated into any of many possible dosage forms such as, but not limited to, tablets, capsules, gel capsules, liquid syrups, soft gels and suppositories. The compositions of the present invention may also be formulated as suspensions in aqueous, non-aqueous or mixed media. Aqueous suspensions may further contain substances, which increase the viscosity of the suspension including, for example, sodium carboxymethylcellulose, sorbitol and/or dextran. The suspension may also contain stabilizers.

For parenteral, subcutaneous, intradermal or topical administration the formulation may include a sterile diluent, buffers, regulators of tonicity and antibacterials. The active compound may be prepared with carriers that protect against degradation or immediate elimination from the body, including implants or microcapsules with controlled release properties. For intravenous administration the preferred carriers are physiological saline or phosphate buffered saline.

An oligonucleotide of the invention may be mixed with any material that do not impair the desired action, or with material that supplement the desired action. These could include other drugs including other nucleoside compounds.

Optionally, the pharmaceutical according to the invention comprises therapeutic agents, such as further antisense compounds, chemotherapeutic compounds, anti-inflammatory compounds, antiviral compounds and/or immuno-modulating compounds. Anti-inflammatory drugs, including but not limited to nonsteroidal anti- inflammatory drugs and corticosteroids, antiviral drugs, and immuno-modulating drugs may also be combined in compositions of the invention.

Two or more combined compounds may be used together or sequentially, *i.e.* the compound according to the invention may be used prior to, during or subsequent to one or more of the other therapeuticagents referred to herein.

Oligonucleotides of the invention may also be conjugated to active drug substances, for example, aspirin, ibuprofen, a sulfa drug, an antidiabetic, an antibacterial or an antibiotic. Preferably, the pharmaceutical composition according to the invention further comprises at least one chemotherapeutic agent. Said chemotherapeutic agent is preferably selected from the group consisting of adrenocorticosteroids, such as prednisone, dexamethasone or decadron; altretamine (hexalen, hexamethylmelamine (HMM)); amifostine (ethyol); aminoglutethimide (cytadren); amsacrine (M-AMSA); anastrozole (arimidex); androgens, such as testosterone; asparaginase (elspar); bacillus calmette-gurin; bicalutamide (casodex); bleomycin (blenoxane); busulfan (myleran); carboplatin (paraplatin); carmustine (BCNU, BiCNU); chlorambucil (leukeran); chlorodeoxyadenosine (2-CDA, cladribine, leustatin); cisplatin (platinol); cytosine arabinoside (cytarabine); dacarbazine (DTIC); dactinomycin (actinomycin-D, cosmegen); daunorubicin (cerubidine); docetaxel (taxotere); doxorubicin (adriomycin); epirubicin; estramustine (emcyt); estrogens, such as diethylstilbestrol (DES); etopside (VP-16, VePesid, etopophos); fludarabine (fludara); flutamide (eulexin); 5-FUDR (floxuridine); 5-fluorouracil (5-FU); gemcitabine (gemzar); goserelin (zodalex); herceptin (trastuzumab); hydroxyurea (hydrea); idarubicin (idamycin); ifosfamide; IL-2 (proleukin, aldesleukin); interferon alpha (intron A, roferon A); irinotecan (camptosar); leuprolide (lupron); levamisole (ergamisole); lomustine (CCNU); mechlorathamine (mustargen, nitrogen mustard); melphalan (alkeran); mercaptopurine (purinethol, 6-MP); methotrexate (mexate); mitomycin-C (mutamucin); mitoxantrone (novantrone); octreotide (sandostatin); pentostatin (2-deoxycoformycin, nipent); plicamycin (mithramycin, mithracin); prorocarbazine (matulane); streptozocin; tamoxifin (nolvadex); taxol (paclitaxel); teniposide (vumon, VM-26); thiotepa; topotecan (hycamtin); tretinoin (vesanoid, all-trans retinoic acid); vinblastine (valban); vincristine (oncovin) and vinorelbine (navelbine).

In a certain embodiments, the present invention provides pharmaceutical compositions containing (a) one or more antisense compounds and (b) one or more other chemotherapeutic agents which function by a non-antisense mechanism. When used with the compounds of the invention, such chemotherapeutic agents may be used individually (e.g. mithramycin and oligonucleotide), sequentially (e.g. mithramycin and oligonucleotide for a period of time followed by another agent and oligonucleotide), or in combination with one or more other such chemotherapeutic agents or in combination with radiotherapy. All chemotherapeutic agents known to a person skilled in the art are here incorporated as combination treatments with compound according to the invention.

In another embodiment, compositions of the invention may contain one or more antisense compounds, particularly oligonucleotides, targeted to a first nucleic acid and one or more additional antisense compounds targeted to a second nucleic acid target. Two or more combined compounds may be used together or sequentially. *i.e*. the compound according to the invention may be used prior to, during or subsequent to one or more of the other therapeutic agents referred to herein.

The pharmaceutical composition of the invention may constitute a pro-drug. Therefore, in some embodiments of the invention the compound of the invention may be in the form of a pro-drug. Oligonucleotides are by virtue negatively charged ions. Due to the lipophilic nature of cell membranes the cellular uptake of oligonucleotides are reduced compared to neutral or lipophilic equivalents. This polarity "hindrance" can be avoided by using the pro-drug approach (see e.g. Crooke, R. M. (1998) in Crooke, S. T. Antisense research and Application. Springer-Verlag, Berlin, Germany, vol. 131, pp. 103-140). In this approach the oligonucleotides are prepared in a protected manner so that the oligo is neutral when it is administered. These protection groups are designed in such a way that so they can be removed then the oligo is taken up be the cells. Examples of such protection groups are S-acetylthioethyl (SATE) or S-pivaloylthioethyl (t-butyl-SATE). These protection groups are nuclease resistant and are selectively removed intracellulary.

Preferably the pharmaceutical composition of the invention further comprises anti-inflamatory compounds and/or antiviral compounds.

In a preferred embodiment, the LNA antisense anti microRNA compounds used in the invention are formulated in saline.

### Specific embodiments

1. A pharmaceutical composition comprising an effective dosage of at least one anti miR-134 oligonucleotide for administration to a mammal suffering from a disease wherein lowering of the activity of miR-134 is beneficial, wherein the time interval between at least two successive administrations is at least 50 days.
2. The pharmaceutical composition of embodiment 1, wherein the successive administrations are each time single administrations.
3. The pharmaceutical composition of any one of embodiment 1 or 2 wherein the time interval between at least two successive administrations, or between each administration is at least 70 days.
4. The pharmaceutical composition according to any one of embodiment 1 or 2, wherein the time interval between at least two successive administrations, or between each administration is at least 90 days.
5. The pharmaceutical composition according to any one of embodiment 1 or 2, wherein the time interval between at least two successive administrations, or between each administration is at least 120 days.
6. The pharmaceutical composition of any one of embodiments 1 to 5, wherein the anti miR-134 oligonucleotide comprises 7 to 16 contiguous nucleotides complementary to sequence UCUCUCCCUCUGGUCAACCAGUCACAAGGCU or comprises 7 to 22 contiguous nucleotides complementary to the hsa-miR-134 (UGUGACUGGUUGACCAGAGGGG, SEQ ID NO 1).
7. The pharmaceutical composition of embodiment 6, wherein the anti miR-134 oligonucleotide is selected from the group consisting of the following sequences: CCCCTCTGGTCAACCAGTCACA, CGTCTAGCCACCTAG and CCTCTGGTCAACCAGTCAC or variants thereof that have at least 80%, 85%, 90%, 95% or 99% sequence identity therewith.
8. The pharmaceutical composition of any one of embodiments 1 to 7, wherein the anti miR-134 oligonucleotide is incapable of recruiting RNaseH.
9. The pharmaceutical composition of any one of embodiments 1 to 8, wherein the anti miR-134 oligonucleotide, contains at least one LNA nucleotide.
10. The pharmaceutical composition of any one of embodiments 1 to 9, wherein the anti miR-134 oligonucleotide is fully phosphorothioate.
11. The pharmaceutical composition of any of embodiments 1 to 10, wherein the anti miR-134 oligonucleotide is a mixmer.
12. The pharmaceutical composition of embodiment 11, wherein the mixmer's length is 10 to 23 nucleotides.
13. The pharmaceutical composition of any of embodiments 1 to 12, wherein the anti miR-134 oligonucleotide is a totalmer.
14. The pharmaceutical composition of embodiment 13, wherein the totalmer's length is 7 to 10 nucleotides.
15. The pharmaceutical composition of any one of embodiment 1 to 14, wherein the anti miR-134 oligonucleotide contains at least one phosphorothioate and at least one LNA is present and C is 5 methylC.
16. The pharmaceutical composition of any one of embodiment 1 to 12, wherein the anti miR-134 oligonucleotide is 5'-TgGtcAAccAgTcAC-3', wherein capital letters indicate beta-D-oxy LNA, lower case DNA and LNA C is 5 methylC and wherein it is a fully phosphorothioate oligonucleotide.
17. The pharmaceutical composition of any one of embodiment 1 to 16, wherein the anti miR-134 oligonucleotide is administered at about 0.05 mg/kg to about 0.15 mg/kg.
18. The pharmaceutical composition of any one of embodiment 1 to 17, wherein the anti miR-134 oligonucleotide is administered at about 0.1 mg/kg.
19. The pharmaceutical composition according to any one of embodiments 1 to 18, wherein at least one of the nucleotide analogues is chosen from the group consisting of: 2'-O-alkyl-RNA monomers, beta-D-oxy-LNA, 6'methyl beta-D-oxy LNA and ENA.
20. The pharmaceutical composition according to any one of embodiments 1 to 19, wherein the anti miR-134 contains a nucleotide analogue which is a locked nucleic acid (LNA).
21. A method of treatment of a mammal suffering from pre-existing epilepsy comprising administering a pharmaceutical composition according to any one of embodiments 1 to 20.
22. The method of embodiment 21, wherein the pharmaceutical composition is administered as a single injection and wherein seizure suppression reaches at least 50% and lasts for at least 50 days after injection.
23. The method of embodiment 21, wherein the pharmaceutical composition is administered as a single injection and wherein seizure suppression reaches at least 50% and lasts for at least 70 days after injection.
24. The method of embodiment 21, wherein the pharmaceutical composition is administered as a single injection and wherein seizure suppression reaches at least 50% and lasts for at least 90 days after injection.
25. The method of embodiment 21, wherein the pharmaceutical composition is administered as a single injection and wherein seizure suppression reaches at least 50% and lasts for at least 120 days after injection.
26. The method or pharmaceutical composition of any one of embodiments 1 to 26 wherein the administration is an injection via intracerebroventricular (ICV).
27. An anti miR-134 oligonucleotide for use in the treatment of a neurological disorder, such as a brain-related disorder characterized by development of seizures, wherein the time interval between two successive administrations is at least 50 days or at least 70, 90 or 120 days.
28. The use of an anti-miR-134 oligonucleotide in the manufacture of a medicament for the treatment of neurological disorder, such as a brain-related disorder characterized by development of seizures, wherein the medicament is for multiple administration to a subject wherein the time interval between two successive administrations is at least about 50 days or at least 70, 90 or 120 days.
29. The use of embodiment 28 wherein multiple administrations are successive single administrations.
30. The anti miR-134 oligonucleotide of embodiment 27 or the use according to embodiment 28 or 29, wherein the anti miR-134 oligonucleotide is as defined in any one of embodiments 1 to 20 or as elsewhere herein.

### Reference List

1. Ambros,V. The functions of animal microRNAs. Nature 431, 350-355 (2004).
2. Bartel,D.P. MicroRNAs: genomics, biogenesis, mechanism, and function. Cell 116, 281-297 (2004).
3. Kloosterman,W.P. & Plasterk,R.H. The diverse functions of microRNAs in animal development and disease. Dev. Cell 11, 441-450 (2006).
4. Soifer,H.S., Rossi,J.J. & Saetrom,P. MicroRNAs in Disease and Potential Therapeutic Applications. Mol Ther (2007)*.*
5. Esau,C. et al. miR-122 regulation of lipid metabolism revealed by in vivo antisense targeting. Cell Metab 3, 87-98 (2006).
6. Krutzfeldt,J. et al. Silencing of microRNAs in vivo with 'antagomirs'. Nature 438, 685-689 (2005).
7. Grimson,A. et al. MicroRNA targeting specificity in mammals: determinants beyond seed pairing. Mol Cell 27, 91-105 (2007).
8. Alvarez-Garcia,I. & Miska,E.A. MicroRNA functions in animal development and human disease. Development 132, 4653-4662 (2005).
9. Abelson,J.F. et al. Sequence variants in SLITRK1 are associated with Tourette's syndrome. Science 310, 317-320 (2005).
10. Calin,G.A. & Croce,C.M. MicroRNA signatures in human cancers. Nat. Rev. Cancer 6, 857-866 (2006).
11. Eisenberg,I. et al. Distinctive patterns of microRNA expression in primary muscular disorders. Proc Natl Acad Sci U S A 104, 17016-17021 (2007).
12. Esquela-Kerscher,A. & Slack,F.J. Oncomirs - microRNAs with a role in cancer. Nat. Rev. Cancer 6, 259-269 (2006).
13. He,L. et al. A microRNA polycistron as a potential human oncogene. Nature 435, 828-833 (2005).
14. Jopling,C.L., Yi,M., Lancaster,A.M., Lemon,S.M. & Sarnow,P. Modulation of hepatitis C virus RNA abundance by a liver-specific MicroRNA. Science 309, 1577-1581 (2005).
15. Lu,J. et al. MicroRNA expression profiles classify human cancers. Nature 435, 834-838 (2005).
16. Pedersen,I.M. et al. Interferon modulation of cellular microRNAs as an antiviral mechanism. Nature 449, 919-922 (2007).
17. Triboulet,R. et al. Suppression of microRNA-silencing pathway by HIV-1 during virus replication. Science 315, 1579-1582 (2007).
18. van Rooij,E. et al. Control of stress-dependent cardiac growth and gene expression by a microRNA. Science 316, 575-579 (2007).
19. Yang,B. et al. The muscle-specific microRNA miR-1 regulates cardiac arrhythmogenic potential by targeting GJA1 and KCNJ2. Nat. Med. 13, 486-491 (2007).
20. Randall,G. et al. Cellular cofactors affecting hepatitis C virus infection and replication. Proc Natl Acad Sci U S A 104, 12884-12889 (2007).
21. Krutzfeldt,J. et al. Specificity, duplex degradation and subcellular localization of antagomirs. Nucleic Acids Res 35, 2885-2892 (2007).
22. Elmen,J. et al. Antagonism of microRNA-122 in mice by systemically administered LNA-antimiR leads to up-regulation of a large set of predicted target mRNAs in the liver. Nucleic Acids Res (2007).
23. Lim,L.P. et al. Microarray analysis shows that some microRNAs downregulate large numbers of target mRNAs. Nature 433, 769-773 (2005).
24. Gentleman,R.C. et al. Bioconductor: open software development for computational biology and bioinformatics. Genome Biol 5, R80 (2004).
25. Lewis,B.P., Burge,C.B. & Bartel,D.P. Conserved seed pairing, often flanked by adenosines, indicates that thousands of human genes are microRNA targets. Cell 120, 15-20 (2005).

### Examples

### Investigate the therapeutic effect of mir-134 antagomir treatment on established spontaneous seizures using EEG recordings in an adult rodent model.

The Henshall lab has shown that adult mice (20 - 25 g) injected with kainic acid (KA) in the amygdala will trigger *status epilepticus* (SE) acutely and spontaneous, recurrent seizures 3-5 days post *status epilepticus.* At 2 weeks post *status epilepticus,* the mice exhibit stable daily epileptic seizures and the seizures continue to occur indefinitely. While the previous work provides a rational for preventative treatment of seizures, the aim was to demonstrate therapeutic efficacy of treatment with mir-134 antagomirs in a state of spontaneous seizure. Adult mice were outfitted with telemetry EEG recording system (vEEG) for chronic monitoring in home cages. On day (0) mice (up to n=6/group) underwent intra-amygdala KA-induced SE, recorded by vEEG. After KA-SE, the mice returned to their home cage and the telemetry units were deactivated. Seven (7) days after induction of KA-SE the telemetry units were turned back on for monitoring of baseline epileptic activity. Fourteen (14) days after KA-SE, the mice were randomly assigned to one of the following three groups: (1) saline/aCSF; (2) Exiqon antagomir; (3) antagomir provided by Roche Innovation Center Copenhagen. The compounds were provided blind to the investigators as "A", "B" and "C". The recommended dose was 0.5 nmol/2µl, one time ICV delivery. After treatment, the mice were continuously monitored by vEEG for the following two (2) weeks. After two (2) weeks of recording, the unit was turned off for four (4) weeks, and then turned back on for an additional two (2) weeks of vEEG recording. The completion of the experiment the mice were sacrificed and the brains removed for future histology and in situ analysis.

A second workpackage was proposed to investigate the impact of early-life seizures on mir-134 levels, and determine effects of preemptive treatment with mir-134 antagomirs on chemo-induced seizures in young rodents. This would aim to confirm that mir-134 is present during juvenile brain development and could be a relevant target. Furthermore, to observe if the expression of mir-134 is regulated upon seizure induction in young rodents. Short-term surface and/or depth EEG recordings would be used to observe seizures in rodents pretreated (24 h) with a mir-134 antagomir or non-targeting (NT) control LNA, administered ICV, to determine the preventative effects on seizure activity. Results of this workpackage will be reported elsewhere.

### Methods

### Animal model and vEEG recordings

All animal experiments were performed in accordance with the European Communities Council Directive (86/609/EEC) and were reviewed and approved by the Research Ethics Committee of the Royal College of Surgeons in Ireland, under license from the Health Research Products Authority and Department of Health, Dublin, Ireland.

Adult male C57BL/6 mice (~25 g) were obtained from RCSI's biomedical research facility (original stock from Harlan, Oxon, Bicester, U.K.). Animals were kept on a 12 hr. light-dark cycle and provided food and water *ad libitum.* Under isoflurane anaesthesia and using aseptic technique, mice were placed in a mouse-adapted stereotaxic frame. A mid-line incision was performed in the skin overlying the skull and Bregma located. Four partial and one complete craniotomy was performed using a microsurgical drill. The leads from a DSI telemetry unit (Model: F20-EET, Data Systems International - DSI) were affixed to the skull for bi-lateral EEG recordings (Ponemah v6.30 software, DSI) and the unit placed in a subcutaneous pocket created in the upper back. A guide cannula was affixed (resting on the dura mater) for the intra-amygdala injection (coordinates: AP = -0.9 mm, L = -2.75 mm from the Bregma). The electrode-cannula assembly was fixed in place by dental cement and the mouse was removed from the stereotaxic frame and allowed to recover from anaesthesia. Mice were returned to home cages and provided soft food and fluids to aid recovery.

Forty-eight hours later, under vEEG monitoring, *status epilepticus* (SE) was triggered in freely-moving awake mice by intra-amygdala microinjection of KA (0.3 µg in 0.2µl) (Sigma-Aldrich, Ireland). After 40 min, all mice received lorazepam (8 mg/kg, i.p.) to curtail seizures and reduce morbidity and mortality. vEEG was recorded for 24 hr. to ensure all mice underwent similar SE, and telemetry units were turned off. Seven days later the EEG telemetry units were activated and continuous EEG recorded with video monitoring in the home cage to obtain an "epileptic baseline". On the 14^{th} day after *status epilepticus,* mice were injected with 0.5 nmol in 2 µl of either compound A, B or C (ICV). Recordings were continued for the following two weeks ("activation period 1") and then interrupted for 4 weeks to conserve telemetry device battery life (battery lasts in total 6 weeks). On the 57^{th} day after *status epilepticus* recordings were re-activated for further two weeks ("activation period 2"). At the end of the entire experiment, mice were deeply anaesthetised and perfused through the heart with cold-buffered saline. Brains were removed for future histology analysis.

### Data analysis

Epileptiform EEG during *status epilepticus* and spontaneous recurrent seizures were analyzed using Neuroscore software (DSI). Data was quantified and Poisson regression and Zero-inflated Poisson regression were used to examine differences in the number of spontaneous seizures in the baseline and activation 1/2 periods, respectively. Deaths during the trial were analysed by Wilcoxon (Breslow) test. Significance was accepted at P < 0.05. Data are presented as mean ± standard error of the mean.

### Results

### Duration of status epilepticus:

There were no differences in the severity of *status epilepticus* between the three groups. Thus, overall, each treatment group experienced a similar severity insult to induce epilepsy.

### Spontaneous seizures in each group during "epileptic baseline" recordings (D07 to D13):

Poisson regression, with robust standard errors to account for clustering of data within animals was used to examine baseline differences. Baseline values were calculated up to but not including day 14. Animals in "C" group had a higher incidence of seizures during the baseline phase (Incidence rate ratio 1·45, P=0·037; see Figure 1). Therefore, the possible confounding influence of baseline seizure rate was next explored. Baseline seizure rate did not predict seizure rate at treatment (activation periods 1 or 2) independently of treatment allocation (p=0·794).

### Spontaneous seizures in each group during "activation period 1" (D15 - D28):

Day 14 was considered the injection day. Treatment phase (activation period 1) was defined as day 15 onward. Both on-drug groups (A and C) had seizure-free days (see raw data appendix), and examination of score distribution revealed a clustering of zero values in animals in each of the on-drug groups. Zero-inflated Poisson regression was used to examine differences in seizures. Incidence of spontaneous seizures was lower in each of the drug treated groups (Drug A, IRR = .22, Drug C IRR = .21, both P < 0·001) in comparison to the control (group B). Therefore, converting IRR means that Group A and C mice had 78% and 79% less spontaneous seizures, respectively, in comparison with group B (control) in the "activation period 1" (see Figure 1). Post hoc tests showed no significant difference in the effects of the drugs A or C (Chi-squared = 0·69, P = 0-402).

It is important to outline that the trial outcomes included both death and seizure counts; and some of the mice presented seizure-free days. Therefore, a score system was developed that allowed these outcomes to be compared in all animals. For this, animals were ranked as follows: (1). Animals who had died were deemed to have a worse outcome than animals who survived. (2). Within decedent animals, length of survival was taken as the measure of outcome, with the worst outcome score attached to the animal with the shortest survival. (3). Within surviving animals, mean seizures per day over the follow-up period ("activation 2") was taken as the measure of outcome, with the worst score assigned to the animal with the highest mean seizure rate. (4). Where animals' scores were tied, the mean rank was assigned. Therefore, when the second analysis has been performed, the differences between groups were ratified (Group A and C mice had 78% and 79% less spontaneous seizures, respectively, in comparison with group B), and again no statistical differences between groups A and C were observed (Figure 1).

Figure 2 shows the total number of seizures per day for experimental groups A (n=4), B (n=6) and C (n=5) for the "epileptic baseline" and "activation 1" periods. **P < 0.001.

### 4.4. Spontaneous seizures during activation Period 2 (D57 - D70):

The follow-up phase (activation period 2) was defined as day 57 onward. Both on-drug groups (A and C) had several seizure-free days (see raw data appendix). Again, the zero-inflated Poisson regression was used, and there is a significantly lower incidence of seizures in the drug treated animals (A and C) on follow-up (Drug A IRR 0·03, Drug C IRR 0·08, both P ≤ 0·001). Therefore, the conversion of IRR for "activation period 2" means that Group A presented 97% of reduction and Group C presented 92% of reduction in the number of spontaneous seizures in comparison with control (B) (see Figure 2). Again, the overall post hoc analysis showed that the effects of the drugs A and C do not differ significantly (Chi-squared = 0·44, P = 0·506). However, when the second analysis has been performed (using the ranking score system) a statistical difference between Groups A and C was verified in the activation 2 period (Group A treatment is slightly more potent than group C; P=0.044). Finally, as expected the differences between treatment groups versus control were ratified (Group A and C mice had 97% and 92% less spontaneous seizures, respectively, in comparison with group B).

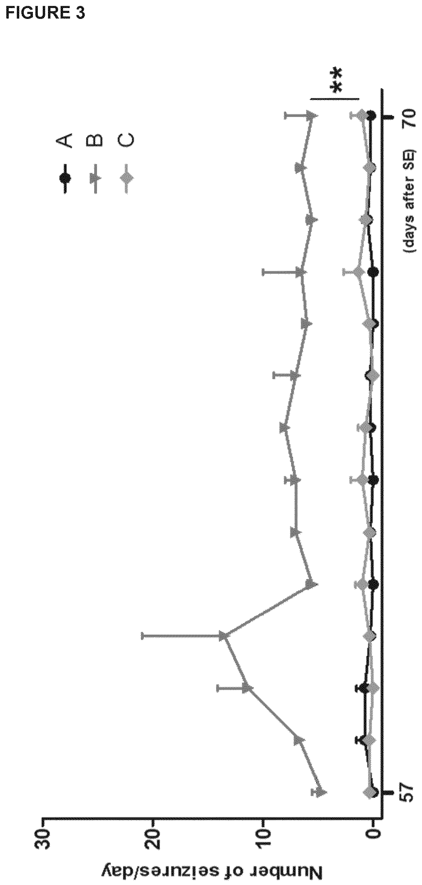

Figure 3 shows the overall analysis of the total number of seizures per day for experimental groups A (n=4), B (n=3) and C (n=3) in the "activation period 2". **P < 0.001.

### Other incidental findings:

*Mortality rate:* When mortality rate was analysed over the whole period by Wilcoxon (Breslow) test comparing either A or C with group B, it was verified that Groups A and C presented lower mortality rate than Group B (borderline statistical significance: P=0·0516).

*Background EEG:* When EEGs were quantified it was observed that the spontaneous seizures in mice from groups A and C seem milder than those from group B (see Figure 3). Further analysis are necessary to measure parameters such as spontaneous seizure duration and ictal severity/power. Furthermore, it was observed that the inter-ictal activity (period between seizures) in mice from groups A and C is similar to a resting EEG background of a normal (non-epileptic) mouse.

Figure 4 shows representative EEG recordings traces showing differences in seizure severity between Ant-134 (e.g. group A; grey tag) and control (red tag) groups. Figure (A-B) represents the occurrence of spontaneous seizures and the inter-ictal activity recording during a normal epileptic baseline period. Representative traces for "activation 1" (C-F) and "activation 2" (G-J) were obtained on the last day of recording of each respective period.

## Claims

1. An anti miR-134 oligonucleotide for use in the treatment of a brain-related disorder **characterized by** development of seizures, wherein the brain related disorder **characterized by** the development of seizures is epilepsy, and wherein the time interval between two successive administrations is 50 days.

2. The anti miR-134 oligonucleotide for use according to claim 1, wherein the successive administrations are each time single administrations.

3. The anti miR-134 oligonucleotide for use according to claim 1 or 2, wherein the anti miR-134 oligonucleotide comprises 7 to 16 contiguous nucleotides complementary to sequence UCUCUCCCUCUGGUCAACCAGUCACAAGGCU or comprises 7 to 22 contiguous nucleotides complementary to the hsa-miR-134 (UGUGACUGGUUGACCAGAGGGG, SEQ ID NO 1).

4. The anti miR-134 oligonucleotide for use according to any of claim 1 to 3, wherein the anti miR-134 oligonucleotide is selected from the group consisting of the following sequences: CCCCTCTGGTCAACCAGTCACA, CGTCTAGCCACCTAG and CCTCTGGTCAACCAGTCAC or variants thereof that have at least 80%, 85%, 90%, 95% or 99% sequence identity therewith.

5. The anti miR-134 oligonucleotide for use according to any of claim 1 to 4, wherein the anti miR-134 oligonucleotide is incapable of recruiting RNaseH.

6. The anti miR-134 oligonucleotide for use according to any of claim 1 to 5, wherein the anti miR-134 oligonucleotide contains at least one LNA nucleotide.

7. The anti miR-134 oligonucleotide for use according to any of claim 1 to 6, wherein the anti miR-134 oligonucleotide is fully phosphorothioate.

8. The anti miR-134 oligonucleotide for use according to any of claim 1 to 7, wherein the anti miR-134 oligonucleotide is a mixmer.

9. The anti miR-134 oligonucleotide for use according to claim 8, wherein the mixmer's length is 10 to 23 nucleotides.

10. The anti miR-134 oligonucleotide for use according to any of claim 1 to 7, wherein the anti miR-134 oligonucleotide is a totalmer

11. The anti miR-134 oligonucleotide for use according to claim 10, wherein the totalmer's length is 7 to 10 nucleotides.

12. The anti miR-134 oligonucleotide for use according to any of claim 1 to 11, wherein the anti miR-134 oligonucleotide contains at least one phosphorothioate and at least one LNA is present and C is 5 methylC.

13. The anti miR-134 oligonucleotide for use according to any of claim 1 to 9, wherein the anti miR-134 oligonucleotide is 5'-TgGtcAAccAgTcAC-3', wherein capital letters indicate beta-D-oxy LNA, lower case DNA and LNA C is 5 methylC and wherein it is a fully phosphorothioate oligonucleotide.

14. The anti miR-134 oligonucleotide for use according to any of claim 1 to 13, wherein the anti miR-134 oligonucleotide is administered at about 0.1 mg/kg or at 0.2 mg/kg.

15. The anti miR-134 oligonucleotide for use according to any of claim 1 to 15, wherein the administration is an injection via intracerebroventricular (ICV).
